# EUROPEAN PATENT SPECIFICATION

(11) **EP 2 210 618 B1**
(45) Date of publication and mention of the grant of the patent: **02.01.2013**
(21) Application number: 10000769.9
(22) Date of filing: 26.01.2010
(51) Int. Cl.: A61L 2/00, A61L 2/20

(54) **Isolator**
Isolator
Isolateur

(30) Priority: 26.01.2009 JP 2009014765; 30.01.2009 JP 2009020984; 25.12.2009 JP 2009293676
(43) Date of publication of application: 28.07.2010
(73) Proprietor: Panasonic Healthcare Co., Ltd., Ehime 791-0395 (JP)
(72) Inventor: Yokoi, Yasuhiko, osaka 570-8677 (JP)
(74) Representative: Glawe, Delfs, Moll

(56) References cited:
- WO-A2-2005/036151
- WO-A2-2008/116341
- JP-A- 2006 068 122
- US-A- 5 173 258

## Description

The present invention relates to a bio isolator according to the preamble of claim 1.

Document WO2008/116341 discloses a decontamination arrangement according to the preamble of claim 1.

A bio isolator, an isolator for bio research, a biohazard isolator or the like is a system having a sterilized workroom, in which operations that require a sterile environment, such as those involving biologically-derived materials obtained by cell culture, for instance, are performed. The sterile environment meant here is an environment substantially free of dust and germs such that it allows no entry of substances other than the materials used in the work done in the workroom. Among conventionally known technologies for such an environment is an isolator having a sterilization chamber which is connected to a workroom via a door and in which articles to be carried into the workroom are sterilized.

The workroom is equipped with a gas supply port and a gas discharge port. Air outside the isolator is supplied into the workroom through the gas supply port, and the air in the workroom is discharged through a gas discharge port. Generally, the isolator is provided with a particulate trap filter, such as a HEPA (High-Efficiency Particulate Air) filter, at the gas supply port in order to keep a sterile environment for the workroom, and the outside air is supplied into the workroom through the particulate trap filter. Also, a particulate trap filter is provided at the gas discharge port, and the air inside the workroom is discharged outside through the particulate trap filter.

Also, in the isolator, a sterilization treatment is conducted in the workroom and the sterilization chamber with a sterilizing material (sterilizing agent), such as hydrogen peroxide gas, sprayed into them.

Under the circumstances as described above, the present inventors have realized the following problems with the system:

In the above-mentioned sterilization treatment, upon completion of sterilization process, a process of replacing the sterilizing material in the workroom with air is carried out, in which the sterilizing material therein is decomposed by a catalyst while air is sent thereinto. At this time, if there is the sterilizing material adsorbed by the particulate trap filter, the replacement process will take a long time because it is not easy to remove the sterilizing material from the particulate trap filter. As a consequence, the process of sterilization as a whole takes a long time.

The isolator is of such design that work in the workroom cannot be started until the concentration of the sterilizing material is low enough to have no adverse effect on the articles to be processed. Besides, in the isolator, after the end of one operation in the workroom, a sterilization treatment is generally performed before the start of the next operation. Hence, if the work efficiency in the isolator is to be enhanced, it is necessary to shorten the time required by the sterilization treatment.

It is an object of the present invention to provide a technology that can shorten the time required by the sterilization treatment by an isolator.

The solution according to the invention resides in the features of the independent claims and preferably in those of the dependent claims.

Embodiments will now be described by way of examples only, with reference to the accompanying drawings which are meant to be exemplary, not limiting and wherein like elements are numbered alike in several Figures in which:
FIG. 1 is a schematic diagram showing a structure of an isolator according to a first embodiment of the present invention;
FIG. 2A is a schematic illustration showing a structure of a sterilizing material supply apparatus; and
FIG. 2B is a cross-sectional view taken along the line A-A of FIG. 2A;
FIG. 3 is a schematic diagram showing a structure of an isolator according to a second embodiment of the present invention;
FIG. 4A is a schematic illustration showing a structure of a sterilizing material supply apparatus in an isolator according to a second embodiment of the present invention; and
FIG. 4B is a cross-sectional view taken along the line A-A of FIG. 4A;
FIG. 5 is a timing chart for explaining a method of generating a sterilizing gas in a sterilizing material supply apparatus of an isolator according to a second embodiment of the present invention;
FIG. 6A is a schematic diagram showing a sterilizing supply apparatus according to a first modification;
FIG. 6B is a cross-sectional view taken along the line A-A of FIG. 6A;
FIG. 7A is a schematic diagram showing a sterilizing supply apparatus according to a second modification; and
FIG. 7B is a cross-sectional view taken along the line A-A of FIG. 7A.

The invention will now be described by reference to the preferred embodiments. This does not intend to limit the scope of the present invention, but to exemplify the invention.
Hereinbelow, the embodiments will be described with reference to the accompanying drawings. Note that in each Figure the same reference numerals are given to the same components and the description thereof is omitted as appropriate. Moreover, the embodiments given are for illustrative purposes only and all features and their combination thereof described in the present embodiment are not necessarily essential to the invention.

### (First embodiment)

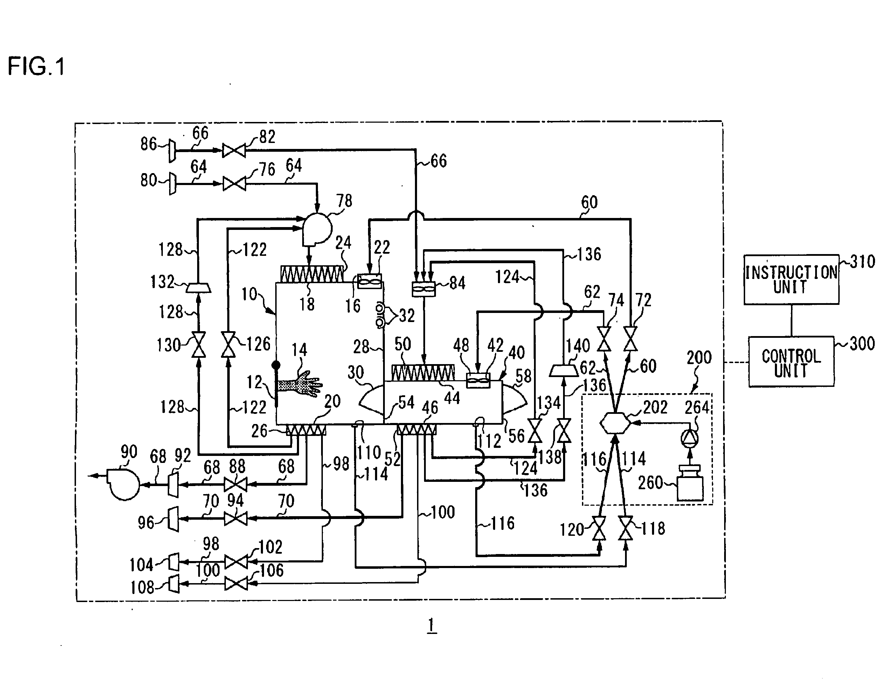

FIG. 1 is a schematic representation showing a structure of an isolator according to a first embodiment of the present invention.

As shown in FIG. 1, an isolator 1 of the first embodiment includes a workroom 10, a sterilization chamber 40, a sterilizing material supply unit 200, and a control unit 300.

The workroom 10 is a space in which work involving biologically-derived materials, such as cell extraction or cell culture, for instance, is performed. The workroom 10 is provided with a front door 12 which is openable and closable, and a work glove 14 to be used in operations in the workroom 10 is installed in a predetermined position of the front door 12. A worker can insert his/her hand into the work glove 14 through a not-shown opening provided in the front door 12 and perform work in the workroom 10 via the work glove 14. Note that biologically-derived materials meant here are materials such as living organisms themselves including cells, substances constituting the living organisms, and substances produced by the living organisms.

The workroom 10 is equipped with a first sterilizing material supply port 16, a first gas supply port 18, and a first gas discharge port 20. The first sterilizing material supply port 16 is provided with a fan 22. The first gas supply port 18 and the first gas discharge port 20 are provided with a HEPA (High-Efficiency Particulate Air) filter 24 and a HEPA filter 26, respectively. It is to be noted that the first sterilizing material supply port 16 is not provided with a HEPA filter. Also, an opening is provided in a side wall 28 of the workroom 10, which is the wall on the side where the sterilization chamber 40 is located, and a door 30 is openably and closably disposed in such a manner as to cover the opening. Further, the workroom 10 is provided with a lighting 32 which illuminates the interior of the workroom 10.

The sterilization chamber 40 is a space in which articles, such as culturing tools, culture media and the like, to be carried into the workroom 10 are sterilized. The sterilization chamber 40 is equipped with a second sterilizing material supply port 42, a second gas supply port 44, and a second gas discharge port 46. The second sterilizing material supply port 42 is provided with a fan 48. Also, the second gas supply port 44 and the second gas discharge port 46 are provided with a HEPA filter 50 and a HEPA filter 52, respectively. It is to be noted that the second sterilizing material supply port 42 is not provided with a HEPA filter. Also, an opening is provided in a side wall 54 of the sterilization chamber 40, which is the wall on the side where the workroom 10 is located, and the sterilization chamber 40 is so disposed that the opening threreof is aligned with the opening provided in the side wall 28 of the workroom 10. Thus the workroom 10 and the sterilization chamber 40 have their internal spaces communicating with each other via a door 30. Further, an opening through which articles are carried into the sterilization chamber 40 is provided in a side wall 56 of the sterilization chamber 40, and a carry-in door 58 is openably and closably disposed in such a manner as to cover the opening.

The sterilizing material supply unit 200 performs a function of supplying a sterilizing material (sterilizing agent) to the workroom 10 and the sterilization chamber 40. The isolator 1 can create a sterile environment in the workroom 10 and the sterilization chamber 40 by supplying the sterilizing material thereinto. Note that the sterile environment meant here is an environment substantially free of dust and germs such that it allows no entry of substances other than the materials used in the work done in the workroom. The sterilizing material supply unit 200 includes a sterilizing gas generator 202, a sterilizing material cartridge 260 for storing the raw material for the sterilizing material, and a pump 264 for supplying the raw material for the sterilizing material from the sterilizing material cartridge 260 to the sterilizing gas generator 202. In the present embodiment, the sterilizing material, or sterilant (sterilizing agent), is a hydrogen peroxide gas, and the raw material for the sterilizing material (sterilizing agent) is a water solution of hydrogen peroxide. It should be noted that the sterilizing material is not limited to hydrogen peroxide gas, but it may be a substance containing active oxygen species such as ozone.

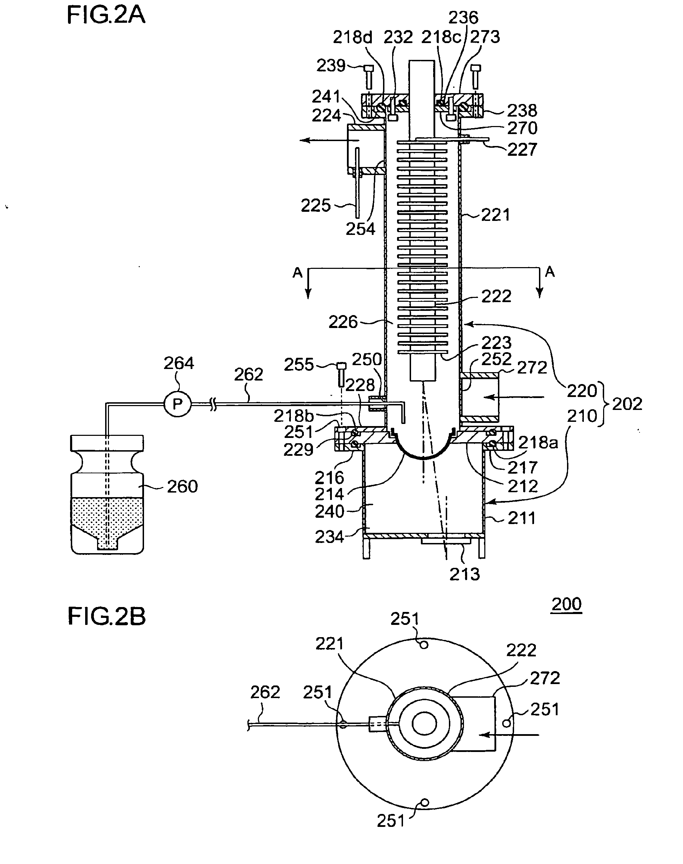

A detailed structure of the sterilizing material supply unit 200 will be explained by referring to FIGS. 2A and 2B. FIG. 2A is a schematic illustration showing a structure of the sterilizing material supply unit 200 according to the first embodiment. FIG. 2B is a cross-sectional view taken along the line A-A of FIG. 2A.

As shown in FIG. 2A, the sterilizing material supply unit 200 includes a sterilizing gas generator 202 which is comprised of a mist generation unit (atomizing unit) 210 and a vaporizing unit 220.

The mist generation unit 210 includes a holding member 211, a partition member 212, an ultrasonic vibrator 213, and a cup 214 serving as the storage unit.

The holding member 211 is a receptacle constituting the mist generation unit 210. The ultrasonic vibrator 213 is disposed on the bottom of the holding member 211. The ultrasonic vibrator 213 is a device that converts electrical energy into ultrasonic vibration. In the present embodiment, the ultrasonic vibrator 213 is not placed in the center of the bottom of the holding member 211 but in a position dislocated closer to an opening 252 to be described later.

A flange 216 is formed around the top periphery of the holding member 211. The top surface of the flange 216 is provided with a groove 217 into which an O-ring 218a is fitted.
The partition member 212 is disposed on top of the flange 216. The partition member 212 is a doughnut-shaped member with an opening formed in the middle thereof. The outside diameter of the partition member 212 is the same as that of the flange 216.
The top surface of the partition member 212 is provided with a groove 229 into which an O-ring 218b is fitted.

The cup 214 is attached to the opening provided in the partition member 212, and the bottom of the cup 214 projects toward the ultrasonic vibrator 213. The cup 214 is made of a resin or with a metal sheet having a thickness of about 0.05 mm, for instance.

A space 234 enclosed by the holding member 211, the partition member 212, and the cup 214 is filled with an ultrasonic propagation liquid 240 which is used to propagate the ultrasonic vibration generated by the ultrasonic vibrator 213. Note that the liquid recommendable as the ultrasonic propagation liquid is water or similar liquid with low viscosity.

In the mist generation unit 210 configured as described above, the hydrogen peroxide solution supplied to the cup 214 is turned into a mist by the ultrasonic vibration propagated through the ultrasonic propagation liquid 240, and the hydrogen peroxide turned into mist is sent into the vaporizing unit 220. In this process, droplets of hydrogen peroxide adhering to the inside of the vaporizing unit 220 without becoming mist will drop to the cup 214 by the force of gravity and will be turned into mist again.

It should be appreciated here that the wavefront direction of the ultrasonic vibration generated by the ultrasonic vibrator 213 is preferably tilting about 7 degrees toward the cup 214. In such an arrangement, the water column by the hydrogen peroxide solution stored by the cup 214 rises up at a tilt, so that a stable atomization of the hydrogen peroxide solution can be accomplished.

The vaporizing unit 220, which is disposed above the mist generation unit 210, is comprised mainly of a heating pipe 221, a heater 222, a piping 224, and thermometers 225 and 227.

The heating pipe 221 is mounted on top of the holding member 211 in such a manner that its axial direction is vertical. To be more specific, the heating pipe 221 has a flange 228 formed around the lower end thereof. The outside diameter of the flange 228 is the same as those of the partition member 212 and the flange 216.

The flange 216, the partition member 212 and the flange 228 are provided with screw holes 251 in predetermined positions. The mist generation unit 210 and the vaporizing unit 220 are assembled by screwing screws 255 into the screw holes 251 with the O-rings 218a and 218b fitted in the grooves 217 and 229, respectively. Airtightness between the flange 216 and the partition member 212 is enhanced by the O-ring 218a. Also, airtightness between the flange 228 and the partition member 212 is enhanced by the O-ring 218b.

The heating pipe 221 has an opening 250 and an opening 252 in the lower part thereof. In the present embodiment, the opening 250 and the opening 252 are located in positions opposite to each other. Led through the opening 250 is the piping 262 which supplies the hydrogen peroxide solution stored in the sterilizing material cartridge 260 to the cup 214. Provided midway along the piping 262 is a pump 264 (e.g., Peristaltic pump) which is used to pump up the hydrogen peroxide solution stored in the sterilizing material cartridge 260.

On the other hand, a piping 272 is connected to the opening 252. And connected to the other end of the piping 272 are the downstream ends of a first circulating path 114 and a second circulating path 116.

Inside the heating pipe 221, a flow path 226 is formed where hydrogen peroxide and air flow from bottom upward.

In the present embodiment, the heating pipe 221 is disposed directly above the cup 214. Therefore, even when the hydrogen peroxide mist recombines into droplets within the heating pipe 221, they will drop down to the cup 214 by the force of gravity. The hydrogen peroxide having returned to the cup 214 will again be turned into a mist by the ultrasonic vibration and sent to the heating pipe 221. Thus, the hydrogen peroxide liquefied in the heating pipe 221 is returned to the cup 214 and turned into mist again by a simple structure, thereby accomplishing a complete gasification of hydrogen peroxide solution in the cup 214.
The heater 222 is disposed inside the heating pipe 221 along its axis. The heater 222 is regulated at a temperature of about 180°C through the ON/OFF control by the control unit 300. The temperature of the heater 222 is detected by the thermometer 227, and the detected temperature is transmitted to the control unit 300. When the temperature of the heater 222 exceeds about 220°C, the control unit 300 cuts off power supply to the heater 222. Note that the heater 222 is preferably provided with a plurality of fins 223. Such an arrangement will promote gasification of hydrogen peroxide by increasing the contact area between the heater 222 and the hydrogen peroxide flowing through the flow path 226.

A flange (fin) 270 for fixation is provided in the upper part of the heater 222. A lid member 273 has a groove 236 formed along the periphery of the heater 222, and an O-ring 218c is fitted in this groove 236. Also, the lid member 273 is provided with an opening through which the upper portion of the heater 222 is inserted. The heater 222 is fastened to the lid member 273 by means of screws 232, and the O-ring 218c enhances airtightness between the flange 270 and the lid member 273.

The heating pipe 221 is provided with a flange 238 at the top portion of the heating pipe 221. The flange 238 has a groove 241 formed in its surface facing the lid member 273, and an O-ring 218d is fitted in this groove 241. The flange 238 is fastened to the lid member 273 by means of screws 239. In this manner, the lid member 273 is mounted on the heating pipe 221 with the O-ring 218d enhancing airtightness, and at the same time the state of the heater 222 inserted in the heating pipe 221 is maintained.

The heating pipe 221 has an opening 254 in a side face of the upper part of the heating pipe 221, to which one end of the piping 224 is connected. Provided on the piping 224 is the thermometer 225 which is used to measure the internal temperature of the piping 224. The internal temperature of the piping 224 measured by the thermometer 225 is transmitted to the control unit 300. Connected to the other end of the piping 224 are the upstream ends of a first sterilizing material supply path 60 and a second sterilizing material supply path 62.

Referring back to FIG. 1, the isolator 1 includes a first sterilizing material supply path 60, a second sterilizing material supply path 62, a first gas supply path 64, a second gas supply path 66, a first gas discharge path 68, and a second gas discharge path 70.

The first sterilizing material supply path 60 connects the sterilizing material supply unit 200 to the first sterilizing material supply port 16 without the medium of a HEPA filter. The first sterilizing material supply path 60 is provided with an on-off valve 72. The sterilizing material generated by the sterilizing material supply unit 200 is supplied into the workroom 10 through the first sterilizing material supply path 60 when the on-off valve 72 is open and the fan 22 is on.

The second sterilizing material supply path 62 connects the sterilizing material supply unit 200 to the second sterilizing material supply port 42 without the medium of a HEPA filter. The second sterilizing material supply path 62 is provided with an on-off valve 74. The sterilizing material generated by the sterilizing material supply unit 200 is supplied into the sterilization chamber 40 through the second sterilizing material supply path 62 when the on-off valve 74 is open and the fan 48 is on.

The first gas supply path 64 connects the exterior of the isolator 1 to the first gas supply port 18. Since the first gas supply port 18 is provided with the HEPA filter 24, the exterior of the isolator 1 and the workroom 10 are connected with each other via the HEPA filter 24. The first gas supply path 64 is provided with an on-off valve 76, and the end on the workroom 10 side of the first gas supply path 64 is connected to a blower 78. The blower 78 is so arranged as to blow gas toward the workroom 10, and a gas, such as air, which exists outside the isolator 1 (hereinafter referred to as "air") is supplied into the workroom 10 through the first gas supply path 64 when the on-off valve 76 is open and the blower 78 is on. Provided at an exterior end of the first gas supply path 64 is a sterilizing material decomposition treatment filter 80 containing a metallic catalyst, such as platinum, which prevents the sterilizing material from flowing back from the workroom 10 and out of the isolator 1.

The second gas supply path 66 connects the exterior of the isolator 1 to the second gas supply port 44. Since the second gas supply port 44 is provided with the HEPA filter 50, the exterior of the isolator 1 and the sterilization chamber 40 are connected with each other via the HEPA filter 50. The second gas supply path 66 is provided with an on-off valve 82, and the end on the sterilization chamber 40 side of the second gas supply path 66 is connected to a fan 84. The fan 84 is so arranged as to send air toward the sterilization chamber 40, and air from outside the isolator 1 is supplied into the sterilization chamber 40 through the second gas supply path 66 when the on-off valve 82 is open and the fan 84 is on. Provided at an exterior end of the second gas supply path 66 is a sterilizing material decomposition treatment filter 86 containing a metallic catalyst, such as platinum, which prevents the sterilizing material from flowing back from the sterilization chamber 40 and out of the isolator 1.

The first gas discharge path 68 connects a first gas discharge port 20 to the exterior of the isolator 1. Since the first gas discharge port 20 is provided with the HEPA filter 26, the workroom 10 and the exterior of the isolator 1 are connected with each other via the HEPA filter 26. The first gas discharge path 68 is provided with an on-off valve 88, and an exterior end of the first gas discharge path 68 is connected to a blower 90. The blower 90 is so arranged as to blow air toward the exterior of the isolator 1, and air inside the workroom 10 is discharged to the exterior of the isolator 1 through the first gas discharge path 68 when the on-off valve 88 is open and the blower 90 is on. Provided between the on-off valve 88 of the first gas discharge path 68 and the blower 90 is a sterilizing material decomposition treatment filter 92 containing a metallic catalyst, such as platinum, which prevents the sterilizing material from flowing from the workroom 10 to the exterior of the isolator 1.

The second gas discharge path 70 connects the second gas discharge port 46 to the exterior of the isolator 1. Since the second gas discharge port 46 is provided with the HEPA filter 52, the sterilization chamber 40 and the exterior of the isolator 1 and are connected with each other via the HEPA filter 52. The second gas discharge path 70 is provided with an on-off valve 94, and air inside the sterilization chamber 40 is discharged to the exterior of the isolator 1 through the second gas discharge path 70 when the on-off valve 94 is open and the fan 84 or the fan 48 is on. Provided at an exterior end of the second gas discharge path 70 is a sterilizing material decomposition treatment filter 96 containing a metallic catalyst, such as platinum, which prevents the sterilizing material from flowing from the sterilization chamber 40 to the exterior of the isolator 1.

Also, the isolator 1 includes a workroom pressure regulating flow path 98 and a sterilization chamber pressure regulating flow path 100.

The workroom pressure regulating flow path 98 connects the first gas discharge port 20 to the exterior of the isolator 1. The workroom pressure regulating flow path 98 is provided with an on-off valve 102, and air inside the workroom 10 is discharged to the exterior of the isolator 1 through the workroom pressure regulating flow path 98 when the on-off valve 102 is open and the blower 78 is on. The flow path diameter of the workroom pressure regulating flow path 98 is set smaller than that of the first gas supply path 64. Accordingly, the supply rate of air into the workroom 10 via the first gas supply path 64 is greater than the discharge rate of air from the workroom 10 via the workroom pressure regulating flow path 98. Therefore, when there is no outflow of air through the first gas discharge path 68 with the on-off valve 88 closed or when the total of the outflow of air through the first gas discharge path 68 and the outflow of air through the workroom pressure regulating flow path 98 is at a prescribed level or below, the pressure in the workroom 10 is maintained positive, thus preventing the entry of foreign matter into the workroom 10 from outside. Provided at an exterior end of the workroom pressure regulating flow path 98 is a sterilizing material decomposition treatment filter 104 containing a metallic catalyst, such as platinum, which prevents the sterilizing material from flowing from the workroom 10 to the exterior of the isolator 1.

The sterilization chamber pressure regulating flow path 100 connects the second gas discharge port 46 to the exterior of the isolator 1. The sterilization chamber pressure regulating flow path 100 is provided with an on-off valve 106, and air inside the sterilization chamber 40 is discharged to the exterior of the isolator 1 through the sterilization chamber pressure regulating flow path 100 when the on-off valve 106 is open and the fan 84 is on. The flow path diameter of the sterilization chamber pressure regulating flow path 100 is set smaller than that of the second gas supply path 66. Accordingly, the supply rate of air into the sterilization chamber 40 via the second gas supply path 66 is greater than the discharge rate of air from the sterilization chamber 40 via the sterilization chamber pressure regulating flow path 100. Therefore, when there is no outflow of air through the second gas discharge path 70 with the on-off valve 94 closed or when the total of the outflow of air through the second gas discharge path 70 and the outflow of air through the sterilization chamber pressure regulating flow path 100 is at a prescribed level or below, the pressure in the sterilization chamber 40 is maintained positive, thus preventing the entry of foreign matter into the sterilization chamber 40 from outside. Provided at an exterior end of the sterilization chamber pressure regulating flow path 100 is a sterilizing material decomposition treatment filter 108 containing a metallic catalyst, such as platinum, which prevents the sterilizing material from flowing from the sterilization chamber 40 to the exterior of the isolator 1.

The isolator 1 according to the present embodiment includes a first sterilizing material discharge port 110 provided in the workroom 10 and a second sterilizing material discharge port 112 provided in the sterilization chamber 40. Also, the isolator 1 includes a circulating path 114 that connects the first sterilizing material discharge port 110 to the sterilizing material supply port 200 without the medium of the HEPA filter and a second circulating path 116 that connects the second sterilizing material discharge port 112 to the sterilizing material supply unit 200 without the medium of the HEPA filter.

An end of the first circulating path 114 on a sterilizing material supply unit 200 side is coupled to the piping 272 (See FIG. 2A). Thus a flow path is formed such that the sterilizing material produced by the sterilizing material supply unit 200 is circulated in the order of the first sterilizing material supply path 60, the workroom 10, the first circulating path 114 and the sterilizing material supply unit 200. The first circulating path 114 is provided with an on-off valve 118. When the on-of valve 118 is open and the fan 22 is on, the sterilizing material in the workroom 10 is sent to the sterilizing supply unit 200 after it has passed through the first circulating path 114.

An end of the second circulating path 116 on a sterilizing material supply unit 200 side is coupled to the piping 272 (See FIG. 2A). Thus a flow path is formed such that the sterilizing material produced by the sterilizing material supply unit 200 is circulated in the order of the second sterilizing material supply path 62, the sterilization chamber 40, the second circulating path 116 and the sterilizing material supply unit 200. The second circulating path 116 is provided with an on-off valve 120. When the on-off valve 120 is open and the fan 48 is on, the sterilizing material in the sterilization chamber 40 is sent to the sterilizing material supply unit 200 after it has passed through the second circulating path 116.

The isolator 1 according to the present embodiment further includes a first filter circulating path 122, which is a first circulating path going through a filter, and a second filter circulating path 124, which is a second circulating path going through a filter.

The first filter circulating path 122 connects the first gas discharge port 20 to the first gas supply port 18, on the outside of the workroom 10. The first filter circulating path 122 is provided with an on-off valve 126. An end of the first filter circulating path 122 on a first gas supply port 18 side is coupled to the blower 78. When the on-off valve 126 is open and the blower 78 is on, the sterilizing material in the workroom 10 is circulated in the order of the first gas discharge port 20, the first filter circulating path 122, the first gas supply port 18 and the workroom 10. At this time, the sterilizing material passes through the HEPA filter 26 and the HEPA filter 24.

The first filter circulating path 122 includes a first decomposition treatment unit path 128 which is a path going through a decomposition treatment unit. The first decomposition treatment unit path 128 connects the first gas discharge port 20 to the first gas supply port 18, on the outside of the workroom 10. An end of the first decomposition treatment unit path 128 on a first gas supply port 18 side is coupled to the blower 78. The first decomposition treatment unit path 128 is provided with an on-off valve 130. Also, the first decomposition treatment unit path 128 is provided with a sterilizing material decomposition treatment filter 132 having a catalyst, such as platinum, which decomposes the sterilizing material. The sterilizing material decomposition treatment filter 132 constitutes a sterilizing material decomposition treatment unit. When the on-off valve 130 is open and the blower 78 is on, the sterilizing material in the workroom 10 is circulated in the order of the first gas discharge port 20, the first decomposition treatment unit path 128, the first gas supply port 18 and the workroom 10. In this process, the sterilizing material passes through the sterilizing material decomposition treatment filter 132 repeatedly so as to be decomposed. As a result, the concentration of the sterilizing material to be discharged outside can be further reduced.

The second filter circulating path 124 connects the second gas discharge port 46 to the second gas supply port 44, on the outside of the sterilization chamber 40. The second filter circulating path 124 is provided with an on-off valve 134. An end of the second filter circulating path 124 on a second gas supply port 44 side is coupled to the fan 84. When the on-off valve 134 is open and the fan 84 is on, the sterilizing material in the sterilization chamber 40 is circulated in the order of the second gas discharge port 46, the second filter circulating path 124, the second gas supply port 44 and the sterilization chamber 40. At this time, the sterilizing material passes through the HEPA filter 52 and the HEPA filter 50.

The second filter circulating path 124 includes a second decomposition treatment unit path 136 which is a path going through a decomposition treatment unit. The second decomposition treatment unit path 136 connects the second gas discharge port 46 to the second gas supply port 44, on the outside of the sterilization chamber 40. An end of the second decomposition treatment unit path 136 on a second gas supply port 44 side is coupled to the blower 84. The second decomposition treatment unit path 136 is provided with an on-off valve 138. Also, the second decomposition treatment unit path 136 is provided with a sterilizing material decomposition treatment filter 140 having a catalyst, such as platinum, which decomposes the sterilizing material. The sterilizing material decomposition treatment filter 140 constitutes a sterilizing material decomposition treatment unit. When the on-off valve 138 is open and the fan 84 is on, the sterilizing material in the sterilization chamber 40 is circulated in the order of the second gas discharge port 46, the second decomposition treatment unit path 136, the second gas supply port 44 and the sterilization chamber 40. In this process, the sterilizing material passes through the sterilizing material decomposition treatment filter 140 repeatedly so as to be decomposed. As a result, the concentration of the sterilizing material to be discharged outside can be further reduced.

The isolator 1 includes the control unit 300. The control unit 300 controls the opening and closing of each on-off valve and the ON/OFF of each blower and fan, and thereby controls the switching of the flow path of sterilizing material and air taken in from the outside. The control unit 300 also controls the generation of sterilizing material by the sterilizing material supply unit 200. The isolator 1 also includes an instruction unit 310 by which a user can select any of various sterilization processes and treatments described later. The control unit 300 has a not-shown storage which stores various kinds of control programs and conversion tables associating each on-off valve and fan with their respective states in each sterilization. The control unit 300 retrieves, from the storage, a control program and a conversion table associated with the sterilization treatment selected by the user, and controls the switching of flow paths according to said control program and conversion tale so as to conduct the selected sterilization treatment.

A description is now given of a sterilization treatment carried out in the isolator 1 configured as above. In a state where a work is being carried out in the workroom 10, the door 30 is closed at first. At this time, the control unit 300 turns on the on-off valves 76, 88 and 102, turns off the on-off valves 118, 126 and 130, and turns on the blower 78 and 90. As a result, the outside air is supplied into the workroom 10 through the first gas supply path 64. This creates a flow path where the air inside the workroom 10 is discharged to the exterior through the first gas discharge path 68 and the workroom pressure regulating flow path 98. The air blowing quantity of the blowers 78 and 90 is adjusted in such a manner that the quantity of air supplied into the workroom 10 from the exterior via the first gas supply path 64 thereof is greater than the quantity of air discharged from the workroom 10 via the first gas discharge path 68 and the workroom pressure regulating flow path 98. Thus the inside of the workroom 10 is maintained at a positive pressure.

A sterilization treatment done by the isolator 1 includes a pretreatment process, a sterilization process, and a replacement process. In the pretreatment process, a sterilizing material is supplied into the workroom 10 or the sterilization chamber 40 which is to be sterilized, so that the workroom 10 or the sterilization chamber 40 is filled with sterilizing material supplied. When the concentration of the sterilizing material in the workroom 10 or the sterilization chamber 40 in the pretreatment process reaches a required concentration level or above, the sterilization process for sterilizing the interior of the workroom 10 or the sterilization chamber 40 starts. As the sterilization process is completed after a predetermined duration of time has elapsed, the replacement process starts. In the replacement process, the sterilizing material is discharged from the workroom 10 or the sterilization chamber 40 and is decomposed by a catalyst, so that the concentration thereof drops. At the same time, the inside of the workroom 10 or the sterilization chamber 40 is replaced with the outside air.

The isolator 1 according to the present embodiment has a first sterilization treatment for sterilizing the interior of the workroom 10 and a second sterilization treatment for sterilizing the interior of the sterilization chamber 40 as the sterilization treatment, and can conduct the first sterilization treatment and the second sterilization treatment independently of each other. Thus, the isolator 1 can sterilize the workroom 10 and the sterilization chamber 40 independently of each other. It is to be noted here that the first sterilization treatment and the second sterilization treatment can be conducted in parallel with each other. In other words, when either one of the sterilization treatments is in progress, the other sterilization treatment can be executed whilst the current sterilization treatment is underway. Note that the second sterilization treatment can be conducted not only when the sterilization chamber 40 is sterilized but also when articles to be carried into the workroom 10 is sterilized.

### (First sterilization treatment: the sterilization of a workroom)

As the control unit 300 receives an instruction to execute the first sterilization treatment from the instruction unit 310, the control unit 300 has the first sterilization treatment conducted. At first, the control unit 300 starts a pretreatment process with the front door 12 and the door 30 closed. In the pretreatment process, the sterilizing material supply unit 200 starts to generate and supply a sterilizing material, and the on-off valves 76, 88, 102, 118, 126 and 130 are closed and the blowers 78 and 90 are turned off. Then, with the on-off valve 72 open and the fan 22 turned on, the sterilizing material is supplied into the workroom 10 by way of the first sterilizing material supply path 60. When the workroom 10 is filled with the sterilizing material and the concentration of the sterilizing material becomes equal to or greater than a concentration level required for the sterilization of the interior of the workroom 10, the pretreatment process is terminated.

As the pretreatment process is terminated, the control unit 300 starts a process of sterilization. In the process of sterilization, the on-off valve 118 is opened and the gas inside the workroom 10 including the sterilizing material is sent to the sterilizing material supply unit 200 via the first circulating path 114. Thus a circulating path is formed such that the sterilizing material is circulated in the order of the first sterilizing material supply path 60, the workroom 10, the first circulating path 114 and the sterilizing material supply unit 200. After a predetermined duration of time has elapsed or the consumption of the raw material for sterilizing material has reached a prescribed level, the process of sterilization is terminated. For example, the process of sterilization may be terminated when the sterilizing material cartridge 260 becomes empty.

As the process of sterilization is terminated, the control unit 300 starts a replacement process. In the replacement process, the sterilizing material supply unit 200 stops the generation and supply of the sterilizing material, and the on-off valve 88 is opened and the blower 90 is turned on. Thus, air inside the workroom 10 including the sterilizing material is discharged to the first gas discharge path 68. Also, the on-off valve 76 is opened and the blower 78 is turned on, so that air outside the isolator 1 is supplied into the workroom 10. In this manner, the sterilizing material in the workroom 10 is replaced by the air. Also, the sterilizing materials in the first sterilizing material supply path 60 and the first circulating path 114 are removed and replaced by the air. The air discharged to the first gas discharge path 68 is discharged outside the isolator 1 by way of the first gas discharge path 68. When the air passes through the sterilizing material decomposition treatment filter 92, the sterilizing material contained in the air is decomposed by the sterilizing material treatment filter 92. Note that the arrangement may be such that the sterilizing material is also discharged to the workroom pressure regulating flow path 98 with the on-off valve 102 open.

When the concentration of the sterilizing material in the workroom 10 becomes a prescribed level or below in the replacement process, the replacement process is terminated and the workroom 10 is ready to be used. Here, the concentration of the sterilizing material for which the workroom 10 can be ready to be used corresponds to a concentration with which a biologically-derived material used in the work is processed without being affected nonnegligibly. If the sterilizing material is a hydrogen peroxide gas, this level of concentration will be less than or equal to 1 ppm time weighted average (TWA) which is specified by the American Conference of Governmental Industrial Hygienists (ACGIH). The concentration of the sterilizing material in the workroom 10 can be obtained if a sensor such as an infrared absorption sensor is installed in the workroom 10. Alternatively, whether the concentration of the sterilizing material in the workroom 10 reaches a prescribed value or not may be detected in a manner such that the time until when the concentration of the sterilizing material in the workroom 10 reaches the prescribed value is calculated and the passage of such time is monitored.

### (Second sterilization treatment: the sterilization of a sterilization chamber)

As the control unit 300 receives an instruction to execute the second sterilization treatment from the instruction unit 310, the control unit 300 has the second sterilization treatment conducted. At first, the control unit 300 starts a pretreatment process with the door 30 and the carry-in door 58 closed and articles being carried into the sterilization chamber 40 if the articles are to be sterilized. In the pretreatment process, the sterilizing material supply unit 200 starts to generate and supply the sterilizing material, and the on-off valves 82, 94, 106, 120, 134 and 138 are closed and the fan 84 is turned off. Then, with the on-off valve 74 open and the fan 48 turned on, the sterilizing material is supplied into the sterilization chamber 40 by way of the second sterilizing material supply path 62. When the sterilization chamber 40 is filled with the sterilizing material and the concentration of the sterilizing material becomes equal to or greater than a concentration level required for the sterilization of the interior of the sterilization chamber 40 or required for the sterilization of the articles carried into the workroom 10.

As the pretreatment process is terminated, the control unit 300 starts a process of sterilization. In the process of sterilization, the on-off valve 120 is opened and the gas inside the sterilization chamber 40 including the sterilizing material is sent to the sterilizing material supply unit 200 via the second circulating path 116. Thus a circulating path is formed such that the sterilizing material is circulated in the order of the second sterilizing material supply path 62, the sterilization chamber 40, the second circulating path 116 and the sterilizing material supply unit 200. After a predetermined duration of time has elapsed or the consumption of the raw material for the sterilizing material has reached a prescribed level, the process of sterilization is terminated. For example, the process of sterilization may be terminated when the sterilizing material cartridge 260 runs out.

As the process of sterilization is terminated, the control unit 300 starts a replacement process. In the replacement process, the sterilizing material supply unit 200 stops the generation and supply of the sterilizing material, and the on-off valve 94 is opened. Thus, air inside the sterilization chamber 40 including the sterilizing material is discharged to the second gas discharge path 70. Also, the on-off valve 82 is opened and the fan 84 is turned on, so that air outside the isolator 1 is supplied into the sterilization chamber 40. In this manner, the sterilizing material in the sterilization chamber 40 is replaced by the air. Also, the sterilizing materials in the second sterilizing material supply path 62 and the second circulating path 116 are removed and replaced by the air. The air discharged to the second gas discharge path 70 is discharged outside the isolator 1 by way of the second gas discharge path 70. When the air passes through the sterilizing material decomposition treatment filter 96, the sterilizing material contained in the air is decomposed by the sterilizing material treatment filter 96. Note that the arrangement may be such that the sterilizing material is also discharged to the sterilization chamber pressure regulating flow path 100 with the on-off valve 106 open.

When the concentration of the sterilizing material in the sterilization chamber 40 becomes a prescribed level or below in the replacement process, the replacement process is terminated and the door 30 or the side wall 56 can be opened, namely, the sterilization chamber 40 is ready to be opened. Here, the concentration of the sterilizing material for which the sterilization chamber 40 is openable corresponds to a concentration with which a worker or a biologically-derived material is not adversely affected even if the sterilizing material is released to the exterior of the isolator 1 or released to the interior of the workroom 10. This level of concentration is less than or equal to 1 ppm time weighted average (TWA) which is specified by the American Conference of Governmental Industrial Hygienists (ACGIH). The concentration of the sterilizing material in the sterilization chamber 40 can be obtained if a sensor such as an infrared absorption sensor is installed in the sterilization chamber 40. Alternatively, whether the concentration of the sterilizing material in the sterilization chamber 40 reaches a prescribed value or not may be detected in a manner such that the time until when the concentration of the sterilizing material in the sterilization chamber 40 reaches the prescribed value is calculated and the passage of such time is monitored.

### (Third sterilization treatment: the sterilization of workroom and sterilization chamber)

The isolator 1 according to the present embodiment has a third sterilization treatment for sterilizing the interior of the workroom 10 and the interior of the sterilization chamber 40 at the same time, as the sterilization treatment. As the control unit 300 receives an instruction to execute the third sterilization treatment from the instruction unit 310, the control unit 300 has the third sterilization treatment conducted. At first, the control unit 300 starts a pretreatment process with the front door 12 and the carry-in door 58 closed and the door 30 open. In the pretreatment process, the sterilizing material supply unit 200 starts to generate and supply a sterilizing material, and the on-off valves 74, 76, 82, 88, 94, 102, 106, 118, 120, 126, 130, 134 and 138 are closed and the blowers 78 and 90 and the fans 48 and 84 are turned off. Then, with the on-off valve 72 open and the fan 22 turned on, the sterilizing material is supplied into the workroom 10 by way of the first sterilizing material supply path 60. The sterilizing material supplied into the workroom 10 is moved into the sterilization chamber 40 through the door 30 and thereby the sterilizing material is supplied to the interiors of the workroom 10 and the sterilization chamber 40. When the concentration of the sterilizing material becomes equal to or greater than concentration levels required for the sterilization of the interiors of the workroom 10 and the sterilization chamber 40, the pretreatment is terminated.

As the pretreatment process is terminated, the control unit 300 starts a process of sterilization. In the process of sterilization, the on-off valve 120 is opened and the gas inside the sterilization chamber 40 including the sterilizing material is sent to the sterilizing material supply unit 200 via the second circulating path 116. Thus a circulating path is formed such that the sterilizing material is circulated in the order of the first sterilizing material supply path 60, the workroom 10, the sterilization chamber 40, the second circulating path 116 and the sterilizing material supply unit 200, thereby sterilizing the workroom 10 and the sterilization chamber 40. After a predetermined duration of time has elapsed or the consumption of the raw material for sterilizing material has reached a prescribed level, the process of sterilization is terminated. For example, the process of sterilization may be terminated when the sterilizing material cartridge 260 becomes empty.

As the process of sterilization is terminated, the control unit 300 starts a replacement process. In the replacement process, the sterilizing material supply unit 200 stops the generation and supply of the sterilizing material, and the on-off valves 88 and 94 are opened and the blower 90 and the fan 84 are turned on. Thus, gas inside the workroom 10 and sterilization chamber 40 including the sterilizing material is discharged to the first gas discharge path 68 and the second gas discharge path 70. Also, the on-off valves 76 and 82 are opened and the blower 78 is turned on, so that air outside the isolator 1 is supplied into the workroom 10 and the sterilization chamber 40. In this manner, the sterilizing material in the workroom 10 and the sterilization chamber 40 is replaced by the air. Also, the sterilizing materials in the first sterilizing material supply path 60 and the second circulating path 116 are removed and replaced by the air. The sterilizing material discharged to the first gas discharge path 68 and the second gas discharge path 70 is decomposed by the sterilizing material decomposition treatment filter 92 and the sterilizing material decomposition treatment filter 96. Note that the arrangement may be such that the sterilizing material is also discharged to the workroom pressure regulating flow path 98 and the sterilization chamber pressure regulating flow path 100 with the on-off valves 102 and 106 open.

When the concentration of the sterilizing materials in the workroom 10 and the sterilization chamber 40 becomes a prescribed level or below in the replacement process, the replacement process is terminated.

Note that the delivery of the sterilizing material into the sterilizing material supply unit 200 via the second circulating path 116 and the delivery of the sterilizing material to thereinto via the first circulating path 114 may be conducted in parallel with each other or in a switchable manner. Hence, the supply rate of the sterilizing material into the workroom 10 can be made to differ from that of the sterilizing material into the sterilization chamber 40, so that the degrees of sterilization can be adjusted in the workroom 10 and the sterilizing chamber 40 separately. Specifically, in the initial stage of the process of sterilization, for instance, the sterilizing material mainly moves from the first sterilizing material supply port 16 toward the first sterilizing material discharge port 110 due to the circulation of the sterilizing material via the first circulating path 114. Accordingly, the main flow of the sterilizing material is so formed as to pass through the workroom 10 only. Then, when the flow of the sterilizing material is switched, after a predetermined duration of time, from the circulation via the first circulating path 114 to the circulation via the second circulating path 116, the sterilizing material mainly moves from the first sterilizing material supply port 16 toward the second sterilizing material discharge port 112. Accordingly, the main flow of the sterilizing material is so formed as to pass through the sterilization chamber 40 also. As a result, the sterilization of interior of the workroom 10 can be conducted in a more concentrated manner.

Alternatively, in the initial stage of the process of sterilization, the main flow of the sterilizing material passing through the workroom 10 and another flow thereof branching out from this main flow into the sterilization chamber 40 are formed in a manner such that the circulation of the sterilizing material via the first circulating path 114 and the circulation thereof via the second circulating path 116 are carried out in parallel with each other. Since the position at which the sterilizing material is supplied is the first sterilizing material supply port 16, the amount of sterilizing material passing through within the workroom 10 is larger than that entering into the sterilization chamber 40. Then, the circulation of the sterilizing material via the first circulating path 114 is shut off after a predetermined duration of time, so that the main flow of the sterilizing material is so formed as to pass through the interior of the sterilization chamber 40. Thus the amount of sterilizing material supplied into the sterilization chamber 40 can be increased as compared with the case when the switching is made between the first circulating path 114 and the second circulating path 116, and the sterilization of the interior of the workroom 10 can be conducted in a more concentrated manner.

Also, in the pretreatment process, the sterilizing material may be supplied into the sterilization chamber 40 via the second sterilizing material supply path 62 and move into the workroom 10 through the door 30. In the process of sterilization, the sterilizing material may be circulated in the order of the second sterilizing material supply path 62, the sterilizing chamber 40, the workroom 10, the first circulating path 114, and the sterilizing material supply unit 200. In such a case, if the circulation of the sterilizing material via the circuit circulating path 114 and the circulation thereof via the second circulating path 116 are conducted in parallel with each other or in a switchable manner, the supply rate of the sterilizing material into the workroom 10 can be made to differ from that of the sterilizing material into the sterilization chamber 40. As a result, the degrees of sterilization can be adjusted in the workroom 10 and the sterilizing chamber 40 separately.

As the sterilizing material passes through the HEPA filter, part of the sterilizing material is adsorbed by the HEPA filter. The sterilizing material adsorbed by he HEPA filter is not easily removed from the HEPA filter and strips off gradually over a comparatively long period of time. Thus, if the sterilizing material remains in the HEPA filter 24 disposed on an air inlet side of the workroom 10, for instance, there is a possibility that the sterilizing material may strip off from the HEPA filter 24 during the work in the workroom 10 and the sterilizing material stripped off may be mixed into the workroom 10. In order to assure the removal of such sterilizing material from the HEPA filter, the time for the replacement process needs to be set longer. In contrast to this, in the above-described first sterilization treatment, the second sterilization treatment and the third sterilization treatment, the sterilizing material is supplied into the workroom 10 and the sterilization chamber 40 without the medium of the HEAP filter 24 disposed on the inlet side of the workroom 10 and the HEPA filter 50 disposed on the inlet side of the sterilization chamber 40. Thus the time for the replacement process can be made shorter as compared with the case where the sterilizing material passes through the HEPA filters. As a result, the time required for the sterilization treatment can be reduced.

### (The sterilization of HEPA filters)

In the above-described first sterilization treatment or the third sterilization treatment, the sterilizing material does not pass through the HEPA filter 24, so that there are cases where the HEPA filter 24 may not be sufficiently sterilized. In the light of this potential problem, the isolator 1 can conduct the following sterilizing treatment to sterilize the HEPA filter 24. This filter sterilization treatment is conducted after either the first sterilization treatment or the third sterilization treatment is selected and the user selects the sterilization treatment of the HEPA filter 24. Also, this filter sterilization treatment may be selected as a fourth sterilization treatment combined with the first sterilization treatment or a fifth sterilization treatment combined with the third sterilization treatment.

As the filter sterilization treatment of the HEPA filter 24 is selected, the control unit 300 has the sterilizing material supplied into the workroom 10 circulated, with predetermined timing, by way of the filter circulating path 122 in the first sterilization treatment or the third sterilization treatment. More specifically, in a sterilization process of the first sterilization treatment or the third sterilization treatment, the on-off valve 126 is opened with predetermined timing and the blower 78 is turned on. This causes the gas including the sterilizing material in the workroom 10 to circulate in the order of the HEPA filter 26, the first filter circulating path 122, the HEPA filter 24 and the workroom 10 so as to sterilize the HEPA filter 24.

Similarly, in the above-described second sterilization treatment or the third sterilization treatment, the sterilizing material does not pass through the HEPA filter 50, so that there are cases where the HEPA filter 50 may not be sufficiently sterilized. In the light of this potential problem, the isolator 1 can conduct the following sterilizing treatment to sterilize the HEPA filter 50. This filter sterilization treatment is conducted after either the second sterilization treatment or the third sterilization treatment is selected and the user selects the sterilization treatment of the HEPA filter 50. Also, this filter sterilization treatment may be selected as a sixth sterilization treatment combined with the second sterilization treatment, a seventh sterilization treatment combined with the third sterilization treatment, or an eighth sterilization treatment combined with the fifth sterilization treatment.

As the filter sterilization treatment of the HEPA filter 50 is selected, the control unit 300 has the sterilizing material supplied into the sterilization chamber 40 circulated, with predetermined timing, by way of the second filter circulating path 124 in the second sterilization treatment or the third sterilization treatment. More specifically, in a sterilization process of the second sterilization treatment or the third sterilization treatment, the on-off valve 134 is opened with predetermined timing and the fan 84 is turned on. This causes the gas including the sterilizing material in the sterilization chamber 40 to circulate in the order of the HEPA filter 52, the second filter circulating path 124, the HEPA filter 50 and the sterilization chamber 40 so as to sterilize the HEPA filter 50.

Furthermore, in the above-described filter sterilization treatment of the HEPA filter 24, the control unit 300 has the sterilizing material supplied into the workroom 10 circulated, with predetermined timing, by way of the first decomposition treatment unit path 128. More specifically, in a sterilization process of the first sterilization treatment or the third sterilization treatment, the sterilizing material is circulated by way of the first filter circulating path 122; then the on-off valve 130 is opened with predetermined timing and the on-off valve 126 is closed. This causes the air including the sterilizing material in the workroom 10 to circulate in the order of the HEPA filter 26, the first decomposition treatment unit path 128, the HEPA filter 24 and the workroom 10, and causes the sterilizing material to repeatedly pass through the sterilizing material decomposition treatment filter 132 so as to be decomposed and removed. As the concentration of the sterilizing material in the workroom 10 becomes a prescribed value or below, the control unit 300 terminates the process of sterilization and starts the replacement process.

Similarly, in the above-described filter sterilization treatment of the HEPA filter 50, the control unit 300 has the sterilizing material supplied into the sterilization chamber 40 circulated, with predetermined timing, by way of the second decomposition treatment unit path 136. More specifically, in a sterilization process of the second sterilization treatment or the third sterilization treatment, the sterilizing material is circulated by way of the second filter circulating path 124; then the on-off valve 138 is opened with predetermined timing and the on-off valve 134 is closed. This causes the air including the sterilizing material in the sterilization chamber 40 to circulate in the order of the HEPA filter 52, the second decomposition treatment unit path 136, the HEPA filter 50 and the sterilization chamber 40, and causes the sterilizing material to repeatedly pass through the sterilizing material decomposition treatment filter 140 so as to be decomposed and removed. As the concentration of the sterilizing material in the sterilization chamber 40 becomes a prescribed value or below, the control unit 300 terminates the process of sterilization and starts the replacement process.

Suppose that the sterilizing material is not subjected to the above-described decomposition and removal prior to the replacement process. Then the decomposition of the sterilizing material in the sterilizing material decomposition treatment filter 92 and the sterilizing material decomposition treatment filter 96 will take a long time. This is because the concentration of the sterilizing material is high in the initial stage of the replacement process. In the process of sterilization according to the present embodiment, on the other hand, the sterilizing material is decomposed after the gas in the workroom 10 passes through the first decomposition treatment unit path 128 repeatedly and the gas in the sterilization chamber 40 passes through the second decomposition treatment unit path 136 repeatedly. Therefore, the sterilizing material in the workroom 10 or the sterilization chamber 40 can be reduced in a shorter time, and consequently the time for sterilization treatment can be shortened. Also, the gas inside the workroom 10 or the sterilization chamber 40 is discharged to the exterior of the isolator 1 after the concentration of the sterilizing material has been sufficiently reduced. Thus, the leak of the sterilizing material to the exterior can be prevented more reliably. Note that the "prescribed value" is the value capable of both reducing the sterilizing material treatment time and preventing the leak of the sterilizing material, for example, and may be set based on the experiments and the results of simulation runs.

When the above-described filter sterilization treatment is conducted, the sterilizing material passes through the HEPA filter 24 and the HEPA filter 50 disposed on the air inlet side. Thus the replacement process takes a longer time than when no filter sterilization treatment is conducted. In the light of this, for example, where one operation has been completed in the workroom and then a sterilization treatment is to be conducted before the start of the next operation, the first sterilization treatment, the second sterilization treatment, or the third sterilization treatment is conducted. Consequently, the time for sterilization treatment can be shortened and the next operation can be started earlier. On the other hand, for example, where one-day-long operation is over and the next operation is scheduled to be conducted the next day, or where periodic maintenance work is to be done, not only the first sterilization treatment, the second sterilization treatment, or the third sterilization treatment but also the the filter steriliziang treatment is conducted. Consequently, the filters disposed on the air inlet side are also sterilized and therefore the reliability of work done in the isolator 1 can be enhanced.

In the first sterilization treatment and the third sterilization treatment, the sterilizing material supplied into the workroom 10 passes through an air-outlet-side HEPA filter 26, so that part of the sterilizing material is adsorbed by the HEPA filter 26. It takes a comparatively long period of time to remove the sterilizing material adsorbed by the HEPA filter 26. However, it is less likely that the sterilizing material flows back into the workroom 10. This is because the sterilizing material stripped off from the HEPA filter 26 is carried along by the flow of air flowing during an operation from the workroom 10 to the exterior of the isolator 1 and is sent out to a first gas discharge path 68 side. Accordingly, if no filter sterilization treatment is conducted, the time for sterilization treatment can be shortened, regardless of whether there is any sterilizing material adsorbed by the HEPA filter 26 or not, when compared with the case where a filter sterilization treatment is conducted.

To sum up the operations of and the advantageous effects achieved by the arrangement thus far explained, the isolator 1 according to the first embodiment is provided with the first sterilizing material supply path 60 which connects the sterilizing material supply unit 200 to the workroom 10 without the medium of a HEPA filter and the second sterilizing material supply path 62 which connects the sterilizing material supply unit 200 to the sterilization chamber 40 without the medium of a HEPA filter. Hence, the interiors of the workroom and the sterilization chamber can be sterilized without the intervention of HEPA filters disposed on the air inlet side of the workroom and the sterilization chamber. This shortens the time required for the sterilization process because the time until the concentration of the sterilizing material reaches a predetermined level in the pretreatment process is shortened and the time for the replacement process is also shortened. Furthermore, the interiors of the workroom and the sterilization chamber can be sterilized more completely because there is reduced wasteful consumption of sterilizing material due to its adsorption to the HEPA filters.

Also, the isolator 1 is provided with the first circulating path 114 which connects the workroom 10 to the sterilizing material supply unit 200 without the medium of a HEPA filter and the second circulating path 116 which connects the sterilization chamber 40 to the sterilizing material supply unit 200 without the medium of a HEPA filter. Hence, the sterilizing material can be sent out to the sterilizing material supply unit without the intervention of HEPA filters disposed on an air outlet side of the workroom and the sterilization chamber. Accordingly, a circulating path can be formed without the intervention of a HEPA filter between the workroom and the sterilizing material supply unit or between the sterilization chamber and the sterilizing material supply unit. This allows sterilization of the interiors of the workroom and the sterilization chamber with improved sterilization efficiency, thus improving the reliability of the isolator.

Also, the isolator 1 is provided with the first gas supply path 64 and the first gas discharge path 68, which connect the exterior and the workroom 10 to each other, and the second gas supply path 66 and the second gas discharge path 70, which connect the exterior and the sterilization chamber 40 to each other. As a result, the sterilizing material inside the workroom and the sterilization chamber can be removed more rapidly and reliably. This will further improve the reliability of the isolator.

Also, the isolator 1 is of such design that the first sterilization treatment, in which the interior of the workroom 10 is sterilized by supplying the sterilizing material thereto via the first sterilizing material supply path 60, and the second sterilization treatment, in which the interior of the sterilization chamber 40 is sterilized by supplying the sterilizing material thereto via the second sterilizing material supply path 62, can be conducted independently of each other. Therefore, it is possible to conduct the sterilization of the workroom and that of the sterilization chamber by staggering their starting hours. For example, a sterilization in the sterilization chamber is started while work is going on in the workroom and if the work in the workroom is completed while the sterilization in the sterilization chamber is still going on, then another sterilization in the workroom can be started without waiting for the completion of sterilization in the sterilization chamber. This will lead to an improvement in the work efficiency in the isolator and an increase in the production of the processed articles.

Also, in the isolator 1, the third sterilization treatment described herein can be conducted. For example, the third sterilization treatment is defined such that, with the door 30 open, the sterilizing material is supplied into the workroom 10 via the first sterilizing material supply path 60 and the sterilizing material having moved from the workroom 10 to the sterilization chamber 40 is sent to the sterilizing material supply unit 200 via the second circulating path 116. Or the third sterilization treatment is defined such that the sterilizing material is supplied into the sterilization chamber 40 via the second sterilizing material supply path 62 and the sterilizing material having moved from the sterilization chamber 40 to the workroom 10 is sent to the sterilizing material supply unit 200 via the first circulating path 114. In this manner, the workroom and the sterilization chamber can be sterilized together. Also, when the sterilizing material is supplied via the first sterilizing material supply path 60, the sterilizing material is sent out via the second circulating path 116, and when the sterilizing material is supplied via the second sterilizing material supply path 62, the sterilizing material is sent out via the first circulating path 114. Thus, the flow of the sterilizing material can be created from the workroom to the sterilization chamber or from the sterilization chamber to the workroom. This helps to accomplish reliable sterilization in both of the workroom and the sterilization chamber.

Also, in the third sterilization treatment of the isolator 1, the sterilizing material is supplied into the workroom 10 and the sterilization chamber 40 via the first sterilizing material supply path 60, and the delivery of the sterilizing material via the second circulating path 116 back to the sterilizing material supply unit 200 and the delivery of the sterilizing material via the first circulating path 114 back to the sterilizing material supply unit 200 are performed in parallel with each other or in a switchable manner. Thus, the supply rate of the sterilizing material into the workroom can be made to differ from that of the sterilizing material into the sterilization chamber, so that the degrees of sterilization can be adjusted in the workroom and the sterilizing chamber separately. Hence, an optimum sterilization treatment can be conducted according to the state of isolator operation.

Also, the isolator 1 is provided with the first filter circulating path 122 which connects the first gas discharge port 20 to the first gas supply port 18 of the workroom 10. In the first sterilization treatment or the third sterilization treatment of the isolator 1, the sterilizing material supplied to the workroom 10 is circulated by way of the first filter circulating path 122 with predetermined timing. Also, the isolator 1 is provided with the second filter circulating path 124 which connects the second gas discharge port 46 to the second gas supply port 44 of the sterilization chamber 40. In the second sterilization treatment or the third sterilization treatment of the isolator 1, the sterilizing material supplied to the sterilization chamber 40 is circulated by way of the second filter circulating path 124 with predetermined timing. Thus the HEPA filters disposed on the air inlet side of the workroom and the sterilization chamber can be sterilized, so that the interiors of the workroom and the sterilization chamber can be more reliably maintained sterile.

Also, the first filter circulating path 122 includes the first decomposition treatment unit path 128 which goes through the sterilizing material decomposition treatment filter 132. And in the first sterilization treatment of the isolator 1, the sterilizing material supplied to the workroom 10 is circulated by way of the first decomposition treatment unit path 128 with predetermined timing. Also, the second filter circulating path 124 includes the second decomposition treatment unit path 136 which goes through the sterilizing material decomposition treatment filter 140. And in the second sterilization treatment of the isolator 1, the sterilizing material supplied to the sterilization chamber 40 is circulated by way of the second decomposition treatment unit path 136 with predetermined timing. As a result, the sterilizing material in the workroom and the sterilization chamber is decomposed by passing through the respective sterilizing material decomposition treatment filters repeatedly. Therefore, the sterilizing material can be reduced in a shorter time, and consequently the time for sterilization treatment can be shortened. Also, the leak of the sterilizing material to the exterior can be prevented more reliably, thus raising the safety of the isolator.

### (Second embodiment)

In the sterilization treatment, a gaseous sterilizing material is supplied into the workroom. In a conventional isolator, when a sterilizing material is sterilized, a long time duration is required until the sterilizing material is turned into a gas. Thus, the overall sterilization process takes a long time, causing a problem of reduced work efficiency in the isolator.

The present embodiment has been made to solve such problems, and a purpose thereof is to provide a technology by which to reduce the time required for a sterilization treatment and enhance the work efficiency in an isolator

A sterilizing material supply unit according to the present embodiment comprises: (1) a mist generation unit including: (i) a propagation fluid holding unit for holding a fluid through which ultrasonic vibration generated by an ultrasonic vibrator attached to a bottom of the holding unit propagates; and a receptacle for holding a hydrogen peroxide solution which is a raw material for sterilizing material, and a (ii) receptacle for holding a hydrogen peroxide solution which is a raw material for sterilizing material, the receptacle being attached onto the propagation fluid holding unit in such a manner as to cover an upper opening of the propagation fluid holding unit, and a bottom of the receptacle projecting toward the ultrasonic vibrator; and (2) a vaporizing unit configured to heat and vaporize the hydrogen peroxide solution atomized by the mist generation unit, wherein the vaporizing unit is installed upright above said mist generation unit and a lower opening thereof communicates with the receptacle, the vaporizing unit including: (i) a carrier gas supply port into which a carrier gas carrying the hydrogen peroxide solution atomized thereby flows through a lower side face of said vaporizing unit; (ii) a heating pipe, disposed on top thereof, having a hydrogen peroxide supply port for supplying hydrogen peroxide, together with the carrier gas, to the exterior; and (iii) a heater disposed inside the heating pipe, wherein the hydrogen peroxide solution atomized by the mist generation unit is fed to the vaporizing unit using the carrier gas, and a hydrogen peroxide gas, vaporized by the vaporizing unit, together with the carrier gas is supplied to the exterior.

By employing this sterilizing material supply apparatus, a two-stage method is implemented where the hydrogen peroxide is atomized (i.e., the generation of mists) by the mist generation unit having the ultrasonic vibrator followed by the vaporization (gasification) thereof by the vaporizing unit. As a result, the gasification of hydrogen peroxide can be conducted promptly. Also, the quantity of heat used in the vaporizing unit can be reduced as compared with the conventional practice. Thus, the time duration required for the gasification of hydrogen peroxide can be shortened and consequently the process of sterilization as a whole can be shortened. As a result, the work efficiency in the isolator can be improved.
Also, an isolator according to the present embodiment has a workroom in which a work involving biologically-derived materials is performed, and the isolator comprises: (1) a mist generation unit including: (i) a propagation fluid holding unit for holding a fluid through which ultrasonic vibration generated by an ultrasonic vibrator attached to a bottom of the holding unit propagates; and a (ii) receptacle for holding a hydrogen peroxide solution which is a raw material for sterilizing material, the receptacle being attached to the propagation fluid holding unit in such a manner as to cover an upper opening of the propagation fluid holding unit, and a bottom of the receptacle projecting toward the ultrasonic vibrator; and (2) a vaporizing unit configured to heat and vaporize the hydrogen peroxide solution atomized by the mist generation unit, the vaporizing unit including: (i) a carrier gas supply port installed upright above the mist generation unit, wherein a lower opening of the carrier gas supply port communicates with the receptacle and a carrier gas carrying the hydrogen peroxide solution atomized thereby flows into a lower side face of the carrier gas supply port; (ii) a heating pipe, disposed on top thereof, having a hydrogen peroxide supply port for supplying hydrogen peroxide, together with the carrier gas, to the exterior; and (iii) a heater disposed inside the heating pipe, wherein the hydrogen peroxide solution atomized by the mist generation unit is fed to the vaporizing unit using the carrier gas, and a hydrogen peroxide gas, vaporized by the vaporizing unit, together with the carrier gas is supplied to the exterior.

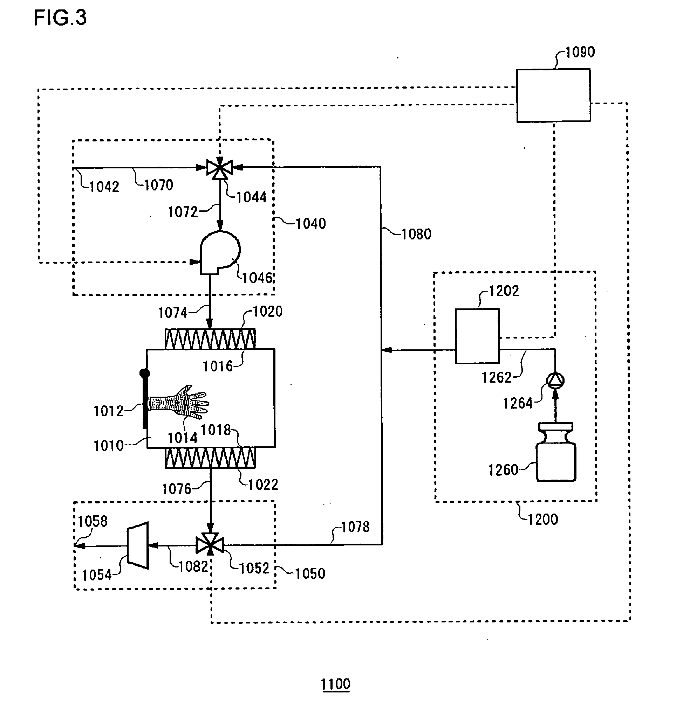

FIG. 3 is a schematic diagram showing a structure of an isolator 1100 according to a second embodiment of the present invention. As shown in FIG. 3, the isolator 1100 of the first embodiment includes a workroom 1010, in which a work involving biologically-derived materials such as cell extraction or cell culture is performed, a gas supply unit 1040 for supplying a gas, a gas discharge unit 1050 for discharging the gas in the workroom 1010, a sterilizing material supply apparatus 1200 for supplying a sterilizing material to the workroom 1010, and a control unit 1090 for controlling these components. Note that biologically-derived materials meant here are materials such as living organisms themselves including cells, substances constituting the living organisms, and substances produced by the living organisms.

The gas supply unit 1040 is provided with an air inlet 1042, a three-way valve 1044, and a fan 1046. Air is taken in from the outside via the air inlet 1042. The three-way valve 1044 is connected downstream of gas flow of the air inlet 1042 via a path 1070 and downstream of gas flow of a sterilizing gas generator 1202 via a path 1080. Also, the three-way valve 1044 is connected upstream of gas flow of the fan 1046 via a path 1072. The three-way valve 1044 is capable of exclusively switching the gas flow path toward either the path 1072 from the path 1070 or the path 1072 from the path 1080. The air taken in via the air inlet 1042 or the gas, including the sterilizing material, sent out via the path 1080 is taken in by the fan 1046 via the three-way valve 1044.

The fan 1046 blows out the gas taken in via the path 1072 from a direction where three-way valve 1044 is disposed, toward a direction where the workroom 1010 is disposed, via a path 1074. The fan 1046 can perform on-off control by the control unit 1090. Note that the fan 1046 can continuously adjust the air volume displacement.

The workroom 1010 is provided with a front door 1012 which is openable and closable.
A work glove 1014 to be used in operations in the workroom 1010 is installed in a predetermined position of the front door 1012. A worker can insert his/her hand into the work glove 1014 through a not-shown opening provided in the front door 1012 and perform work in the workroom 1010 via the work glove 1014. Air sent out from the fan 1046 enters the workroom 1010 from a gas supply port 1016, and is discharged from a gas discharge port 1018. The gas supply port 1016 is provided with a HEPA (High-Efficiency Particulate Air) filter 1020, and the gas supply port 1018 is provided with a HEPA filter 1022. Such an arrangement as described above ensures that the workroom 1010 is maintained sterile. Air flowing out of the workroom 1010 is sent out to the gas discharge unit 1050 by way of he gas discharge port 1018, the HEPA filter 1022 and a path 1076.

The gas discharge unit 1050 is comprised of a three-way valve 1052, a sterilizing material reduction unit 1054 and a discharge port 1058 arranged in this order along the gas flow.

The three-way valve 1052 is connected downstream of gas flow of the workroom 1010 via the path 1076 and upstream of gas flow of the sterilizing material reduction unit 1054 via the path 1082. Also, the three-way valve 1052 is connected upstream of gas flow of the sterilizing material supply apparatus 1200 (described later) via a path 1078. The three-way valve 1052 is capable of exclusively switching the gas flow path toward either the path 1082 from the path 1076 or the path 1078 from the path 1076. The gas taken in via the path 1076 is sent out toward the path 1082 or path 1078.

The sterilizing material reduction unit 1054 reduces the concentration level of the sterilizing material contained in the gas sent out via the three-way valve 1052. The sterilizing material reduction unit 1054 may include a metallic catalyst, such as platinum, and may include an activated carbon or the like.

The sterilizing material supply apparatus 1200 for supplying the sterilizing material to the workroom 1010 is disposed exterior to the workroom 1010. The sterilizing material supply apparatus 1200 can create a sterile environment in the workroom 1010 and the paths by supplying the sterilizing material into the workroom 1010 and circulating it within the isolator 1100. Note that the sterile environment meant here is an environment substantially free of dust and germs such that it allows no entry of substances other than the materials used in the work done in the workroom. In the present embodiment, the sterilizing material, or sterilant (sterilizing agent), is hydrogen peroxide.

As shown in FIG. 3, the sterilizing material supply apparatus 1200 is connected downstream of gas flow of the three-way valve 1052 and the path 1078 and upstream of gas flow of the path 1080 and the three-way valve 1044. The sterilizing material supply apparatus 1200 includes a sterilizing material cartridge 1260, a pump 1264, and a sterilizing gas generator 1202. The sterilizing material cartridges 1260 stores the water solution of hydrogen peroxide as the sterilizing material. The pump 1264 pumps up the hydrogen peroxide solution stored in the sterilizing material cartridge 1260 and sends it out to the sterilizing gas generator 1202. The sterilizing gas generator 1202 vaporizes the hydrogen peroxide solution supplied and generates a hydrogen peroxide gas. The thus generated hydrogen peroxide gas is sent out to the path 1080.

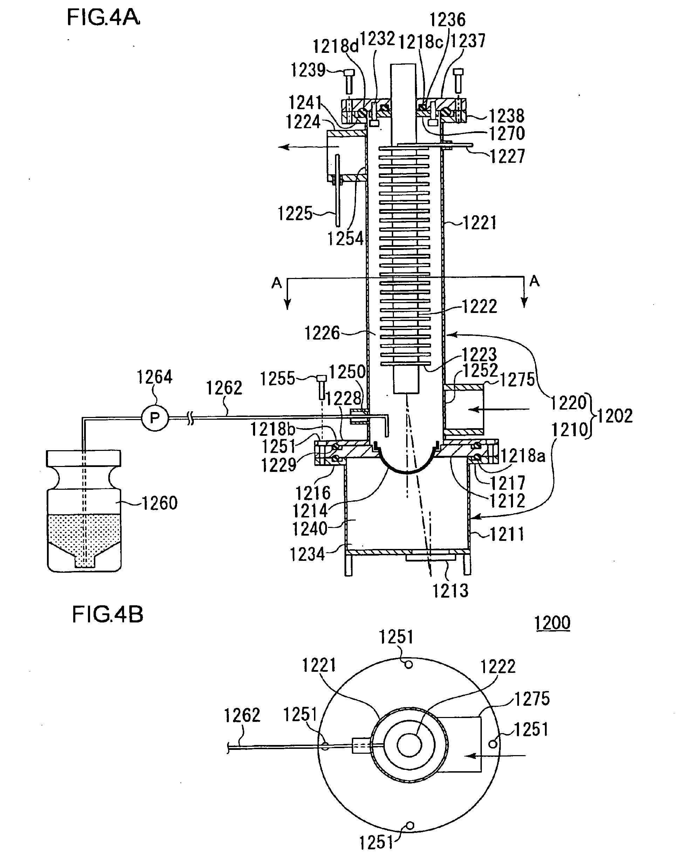

A concrete structure of the sterilizing material supply apparatus 1200 will now be described in detail with reference to FIG. 4A and FIG. 4B. FIG. 4A is a schematic diagram showing a structure of the sterilizing material supply apparatus 1200 according to a first embodiment of the present invention. FIG. 4B is a cross-sectional view taken along the line A-A of FIG. 4A.

As shown in FIG. 4A, the sterilizing material supply unit 1200 includes the sterilizing gas generator 1202 which is comprised of a mist generation unit (atomizing unit) 1210 and a vaporizing unit 1220.

The mist generation unit 1210 includes a holding member 1211, a partition member 1212, an ultrasonic vibrator 1213, and a cup 1214 serving as a receptacle for holding the hydrogen peroxide solution which is a raw material for sterilizing agent.

The holding member 1211 is a propagation fluid holding unit that constitutes the mist generation unit 1210. The ultrasonic vibrator 1213 is disposed on the bottom of the holding member 1211. The ultrasonic vibrator 1213 is a device that converts electrical energy into ultrasonic vibration. In the present embodiment, the ultrasonic vibrator 1213 is not placed in the center of the bottom of the holding member 1211 but in a position dislocated closer to an opening 1252 to be described later.

A flange 1216 is formed around the top periphery of the holding member 1211. The top surface of the flange 1216 is provided with a groove 1217 into which an O-ring 1218a is fitted.

The partition member 1212 is disposed on top of the flange 1216. The partition member 1212 is a doughnut-shaped member with an opening formed in the middle thereof. The outside diameter of the partition member 1212 is the same as that of the flange 1216. The top surface of the partition member 1212 is provided with a groove 1229 into which an O-ring 1218b is fitted.

The cup 1214 is attached to the opening provided in the partition member 1212, and the bottom of the cup 1214 projects toward the ultrasonic vibrator 1213. The cup 1214 is made of a polyester resin, such as PET, having a thickness of about 0.2 mm or of a metal sheet, such as stailess steel, having a thickness of about 0.05 mm, for instance.

A space 1234 enclosed by the holding member 1211, the partition member 1212, and the cup 1214 is filled with an ultrasonic propagation liquid 1240 which is used to propagate the ultrasonic vibration generated by the ultrasonic vibrator 1213. Note that the liquid recommendable as the ultrasonic propagation liquid is water or similar liquid with low viscosity.

In the mist generation unit 1210 configured as described above, the hydrogen peroxide solution supplied to the cup 1214 is turned into a mist by the ultrasonic vibration propagated through the ultrasonic propagation liquid 1240, and the hydrogen peroxide turned into mist is sent into the vaporizing unit 1220. In this process, droplets of hydrogen peroxide adhering to the inside of the vaporizing unit 1220 without becoming mist will drop to the cup 1214 by the force of gravity and will be turned into mist again.

It should be appreciated here that the wavefront direction of the ultrasonic vibration generated by the ultrasonic vibrator 1213, namely the direction normal to the center of the vibration surface of the ultrasonic vibrator 1213, is tilted toward the direction where the lowest part of the cup is located or the direction where the lowest part of the heater 1222 (described later) is located. The direction normal to the center of the vibration surface thereof is preferably tilting about 7 degrees, for example, relative to the vertical direction (i.e., the vertical direction of the mist generation unit 1210). In such an arrangement, the water column by the hydrogen peroxide solution stored by the cup 1214 rises up at a tilt, so that a stable mist generation of the hydrogen peroxide solution can be accomplished.

The vaporizing unit 1220, which is disposed above the atomizing unit 1210, is comprised mainly of a heating pipe 1221, a heater 1222, a piping 1224, and thermometers 1225 and 1227.

The heating pipe 1221 is mounted on top of the holding member 1211 in such a manner that its axial direction is vertical. To be more specific, the heating pipe 1221 has a flange 1228 formed around the lower end thereof. The outside diameter of the flange 1228 is the same as those of the partition member 1212 and the flange 1216.

The flange 1216, the partition member 1212 and the flange 1228 are provided with screw holes 1251 in predetermined positions. The atomizing unit 1210 and the vaporizing unit 1220 are assembled by screwing screws 1255 into the screw holes 1251 with the O-rings 1218a and 1218b fitted in the grooves 1217 and 1229, respectively. Airtightness between the flange 1216 and the partition member 1212 is enhanced by the O-ring 1218a. Also, airtightness between the flange 1228 and the partition member 1212 is enhanced by the O-ring 1218b.

The heating pipe 1221 has an opening 1250 and an opening 1252 in the lower part thereof. In the present embodiment, the opening 1250 and the opening 1252 are located in positions opposite to each other. Led through the opening 1250 is the piping 1262 which supplies the hydrogen peroxide solution stored in the sterilizing material cartridge 1260 to the cup 1214. Provided midway along the piping 1262 is a pump 1264 (e.g., Peristaltic pump) which is used to pump up the hydrogen peroxide solution stored in the sterilizing material cartridge 1260.
On the other hand, connected to the opening 1252 is a piping 1275 into which air (carrier gas) sent out from an air supply fan (not shown) flows, which constitutes a carrier gas supply port. The path 1078 may be connected to the opening 1252. By implementing this arrangement, the sterilizing material gas generator 1202 comprised of the mist generation unit 1210 and the vaporizing unit 1220 functions as a part of the circulating path that goes through the workroom 1010, so that the hydrogen peroxide solution can be supplied to the circulating path as necessary. Inside the heating pipe 1221, a flow path 1226 is formed where hydrogen peroxide and air flow from bottom upward.

In the present embodiment, the heating pipe 1221 is disposed directly above the cup 1214. Therefore, even when the hydrogen peroxide mist recombines into droplets within the heating pipe 1221, they will drop down to the cup 1214 by the force of gravity. The hydrogen peroxide having returned to the cup 1214 will again be turned into a mist by the ultrasonic vibration and sent to the heating pipe 1221. Thus, the hydrogen peroxide liquefied in the heating pipe 1221 is returned to the cup 1214 and turned into mist again by a simple structure, thereby accomplishing a complete gasification of hydrogen peroxide solution in the cup 1214.

The heater 1222 is disposed inside the heating pipe 1221 along its axis. The heater 1222 is regulated at a temperature of about 180°C through the ON/OFF control by the control unit 1300. The temperature of the heater 1222 is detected by the thermometer 1227, and the detected temperature is transmitted to the control unit 1300. When the temperature of the heater 1222 exceeds about 220°C, the control unit 1300 cuts off power supply to the heater 1222. Note that the heater 1222 is preferably provided with a plurality of fins 1223. Such an arrangement will promote gasification of hydrogen peroxide by increasing the contact area between the heater 1222 and the hydrogen peroxide flowing through the flow path 1226.

A flange 1270 for fixation is provided in the upper part of the heater 1222. A lid member 1273 has a groove 1236 formed along the periphery of the heater 1222, and an O-ring 1218c is fitted in this groove 1236. Also, the lid member 1273 is provided with an opening through which the upper portion of the heater 1222 is inserted. The heater 1222 is fastened to the lid member 1273 by means of screws 1232, and the O-ring 218c enhances airtightness between the flange 1270 and the lid member 1273.

The heating pipe 1221 is provided with a flange 1238 at the top portion of the heating pipe 1221. The flange 1238 has a groove 1241 formed in its surface facing the lid member 1273, and an O-ring 1218d is fitted in this groove 1241. The flange 1238 is fastened to the lid member 1273 by means of screws 1239. In this manner, the lid member 1273 is mounted on the heating pipe 1221 with the O-ring 1218d enhancing airtightness, and at the same time the state of the heater 1222 inserted in the heating pipe 1221 is maintained.

The heating pipe 1221 has an opening 1254 in a side face of the upper part of the heating pipe 1221, to which one end of the piping 1224 is connected. This opening 1254 constitutes a hydrogen peroxide supply port for supplying not only the carrier gas but also the hydrogen peroxide to the exterior. Provided on the piping 1224 is the thermometer 1225 which is used to measure the internal temperature of the piping 1224. The internal temperature of the piping 1224 measured by the thermometer 1225 is transmitted to the control unit 1300. Connected to the other end of the piping 1224 are the downstream end of the first sterilizing material supply path 1078 and the upstream end of the second sterilizing material supply path 1080.

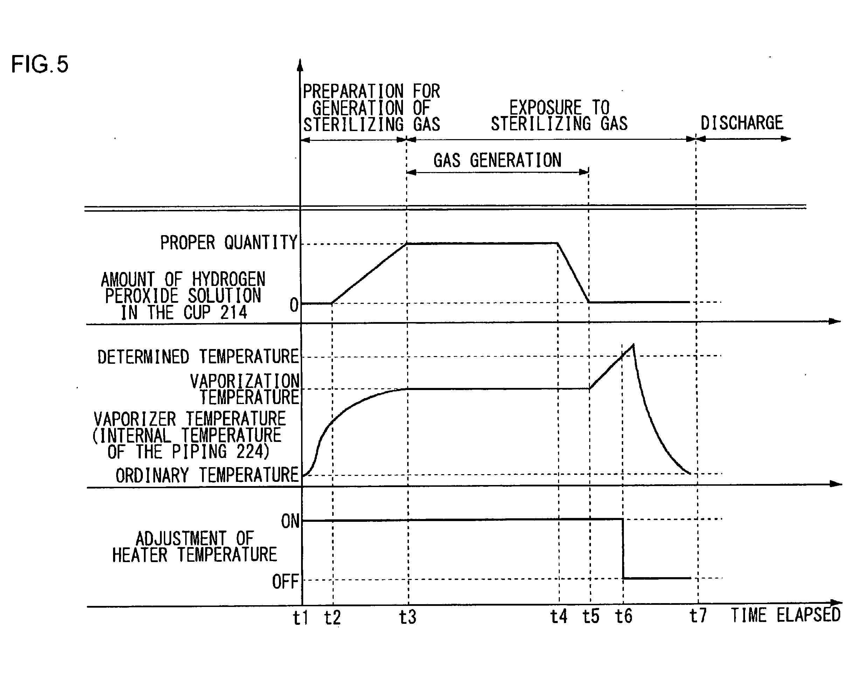

A description is now given of an operation of generating a sterilizing gas in the sterilizing material supply apparatus 1200 structured as above, with reference to the timing charts of FIG. 5. Note that the generation of sterilizing gas by the sterilizing material supply apparatus 1200 is controlled by the control unit 1090.

At time t1, the heater 1222 is first switched on and the heating of the heater 1222 starts. At the same time, the hydrogen peroxide solution stored in the sterilizing material cartridge1 1260 is pumped up by driving the pump 1264, and the hydrogen peroxide solution is delivered toward the cup 1214.

As the heater 1222 is switched on, the internal temperature of the piping1 1224 starts to rise from an ordinary temperature. As, at time t2, the hydrogen peroxide solution reaches the cup 1214 after having passed through the piping 1262, the hydrogen peroxide solution starts to accumulate at the bottom of the cup 1214 and therefore the amount of hydrogen peroxide solution in the cup 1214 starts to rise.

As the internal temperature of the piping 1224 reaches the vaporization temperature of hydrogen peroxide at time t3, the ultrasonic vibrator 1213 starts to be driven and the ultrasonic vibration propagates to the cup 1214 through the ultrasonic propagation fluid 1240. The supply of air starts and therefore the air is sent into the cup 1214 from the outside. As a result, the hydrogen peroxide is turned into a mist in the cup 1214, and the hydrogen peroxide which has been turned into mist is supplied to the heating pipe 1221 by the air sent from the air-supply fan. The hydrogen peroxide in the form of mist supplied to the heating pipe 1221 is heated by the heater 1222 so as to be completely gaseous. The gaseous hydrogen peroxide is supplied to the path 1080 by way of the piping 1224.

In a mist generation apparatus using the ultrasonic vibrator, the mist generation efficiency varies according to the distance between the surface of the ultrasonic vibrator and the liquid level up to which the mist is generated. Thus it is known in the art that an appropriate distance must be maintained. Accordingly, it is desirable that the flow rate at which the hydrogen peroxide solution is delivered to the cup 1214 by the pump 1264 be adjusted in such a manner as to supplement the hydrogen peroxide solution in the cup 1214 consumed along with the progress of mist generation and gasification.
As a result, at time t3 and thereafter, the hydrogen peroxide solution in the cup 1214 can be efficiency turned into mist. The total amount of hydrogen peroxide solution delivered to the cup 1214 is predetermined, so that the drive of the pump 1264 is stopped after a predetermined period of time, which is set according to the capacity of the pump 1264, has elapsed.

As the drive of the pump 1264 has stopped at time t3 or later, the supply of the hydrogen peroxide solution to the cup 1214 is stopped at time t4. After time t4, the amount of hydrogen peroxide solution in the cup 1214 drops gradually and the remaining amount thereof becomes zero at time t5.

After time t5, the amount of hydrogen peroxide in the form of mist sent from the mist generation unit 1210 and the vaporizing unit 1220 drops gradually. Thus the heat quantity released by the vaporization of hydrogen peroxide in the heating pipe 1221 drops gradually. As a result, the internal temperature of the piping 1224 rises further above the vaporization temperature of hydrogen peroxide. In other words, the rise of the internal temperature of the piping 1224 is a phenomenon indicating that the remaining amount of hydrogen peroxide in the cup 1214 becomes zero. Thus, detecting this phenomenon allows one to estimate whether the remaining amount of hydrogen peroxide is zero or not. When the internal temperature of the piping 1224 measured by the thermometer 1225 reaches a predetermined temperature at time t6, the drive of the ultrasonic vibrator 1213 is stopped and the heater 222 is switched off, thereby stopping the heating by the heater 1222. Note that the predetermined temperature corresponds to the internal temperature of the piping 1224 in a state where the gasification of hydrogen peroxide in the heating pipe 1221 has completed and only the air inside the heating pipe 1221 starts to move. That is, the phenomenon that the internal temperature of the piping 1224 reaches the predetermined temperature indicates that the remaining amount of hydrogen peroxide solution in the cup 1214 becomes zero and no hydrogen peroxide solution to be gasified exists in the sterilizing material supply apparatus 1200.

After time t6, the internal temperature of the piping 1224 continues to rise and then drops gradually. At time t7, the internal temperature thereof returns to an ordinary temperature.

The time period from time t1 to time t3 corresponds to a time duration required for the sterilizing gas to be generated. From time t3 to time t4, the mist generation unit 1210 generates the mist from the hydrogen peroxide. From time t1 to time t7, the sterilizing gas is exposed to paths including the workroom 1010 (i.e., a pretreatment process and a sterilization process to be discussed later). After time t7, the sterilizing gas is discharged from the paths including the workroom 1010 (i.e., a replacement process to be discussed later).

A description is now given of the control unit 1090 by referring back to FIG. 3. As described above, the control unit 90 controls the delivery of sterilizing gas by the sterilizing material supply apparatus 1200. Also, the control unit 1090 switches the gas flow path by controlling the opening and closing of valves in the three-way valves 1044 and 1052.

More specifically, the control unit 1090 controls the opening and closing of valves of the three-way valve 1044 and thereby controls the exclusive switching of the gas flow path to either the path 1072 from the path 1070 or the path 1072 from the path 1080. Also, the control unit 1090 controls the opening and closing of valves of the three-way valve 1052 and thereby controls the exclusive switching of the gas flow path to either the path 1082 from the path 1076 or the path 1078 from the path 1076.

### (The switching of gas flow path)

The gas paths in the isolator 1100 are switched as follows in two difference ways when the control unit 1090 controls the opening and closing of the three-way valves 1044 and 1052. That is, when the hydrogen peroxide gas is circulated in the isolator 1100, the three-way valve 1044 is set such that only a passage connecting from the path 1080 to the path 1072 is opened and a passage leading from the path 1070 to the path 1072 is closed. Also, the three-way valve 1052 is set such that only a passage connecting from the path 1076 to the path 1078 is opened and a passage connecting from the path 1076 to path 1082 is closed.

When, on the other hand, the replacement of air in the workroom is to be done, the three-way valve 1044 is set such that only the passage leading from the path 1070 to the path 1072 is opened and the passage leading from the path 1080 to path 1072 is closed. Also, the three-way valve 1052 is set such that only a passage leading from the path 1076 to the path 1082 is opened and the passage leading from the path 1076 to path 1078 is closed. With this configuration described as above, a sequence of paths is formed as followed. Air enters from the air inlet 1042 and flows into the workroom 1010 after passing through the three-way valve 1044, the path 1072, the fan 1046, the path 1074, the HEPA filter 1020 and the gas supply port 1016. Then the air is discharged from the discharge port 1058 after passing through the gas discharge port 1018, the HEPA filter 1022, the path 1076, the three-way valve 1052, the path 1082 and the sterilizing material reduction unit 1054.

### (Sterilization treatment)

In the isolator 1100, after the end of one operation (previous work) in the workroom 1010, a sterilization treatment of the workroom 1010 and the paths used in the previous work is performed before the start of the next operation. The sterilization treatment includes a pretreatment process, a sterilization process, and a replacement process.

In the pretreatment process, the hydrogen peroxide gas is supplied into the workroom 1010 from the sterilizing material supply apparatus 1200, so that the concentration of hydrogen peroxide gas is maintained at a level or above, which is required for the sterilization of the workroom 1010. After the concentration of hydrogen peroxide gas in the workroom 1010 in the pretreatment process has reached a prescribed level or above, the sterilization process starts.

In the process of sterilization, a circulation path is formed such that the hydrogen peroxide gas is sent to the workroom 1010 from the sterilizing material supply apparatus 1200 and is returned again to the sterilizing material supply apparatus 1200. In so doing, the three-way valve 1044 is switched to the open state only in the passage connecting from the path 1080 to the path 1072, and is switched to the closed state in the passage connecting from the path 1070 to the path 1072. On the other hand, the three-way valve 52 is switched to the open state only in the passage connecting from the path 1076 to the path 1078, and is switched to the closed state in the passage connecting from the path 1076 to the path 1082. Thus, a gas flow path is formed in the isolator 1100 such that the gas flows from the three-way valve 1044 into the workroom 1010 and then returns to the three-way valve 1044 via the three-way valve 1052. And the hydrogen peroxide gas introduced into the workroom 1010 can be circulated within the isolator 1100 by controlling the operation of the fan 1046. The HEPA filters 1020 and 1022, the paths 1074 and 1076 and the like can be sterilized, as necessary, by controlling the flow path and the fan in this manner.

In the replacement process, air taken in via the air inlet 1042 is supplied into the workroom 1010 and the gas inside the workroom 1010 is pushed out, so that the gas inside the workroom 1010 is replaced. More specifically, in the replacement process, the three-way valve 1044 is switched to the open state only in a passage connecting from the air inlet 1042 to the workroom 1010, and the three-way valve 1052 is switched to the open state only in a passage connecting from the workroom 1010 to the discharge port 1058. The control unit 1090 turns the fan 1046 on. This forms a gas flow path in the isolator 1100 such that the air taken in from the air inlet 1042 passes through the HEPA filter 1020 from the path 1070, reaches the workroom 1010, then passes through the HEPA filter 1022 from the workroom 1010, and is discharged from the discharge port 1058. As a result, the gas inside the workroom 1010 is replaced by air and therefore the hydrogen peroxide gas in the workroom 1010 is removed from the workroom 1010.

At the same time, the hydrogen peroxide gas pushed out of the workroom 1010 is subjected to a reduction treatment by the sterilizing material reduction unit 1054, so that the outflow of hydrogen peroxide gas from the discharge port 1058 to the exterior is reduced. In the replacement process, the hydrogen peroxide gas staying on within a region other than the workroom 1010 in the isolator 1100, for example, within the gas supply unit 1040 and the hydrogen peroxide adsorbed by the HEPA filters 1020 and 1022 in the flow paths used in the previous work are also removed.

By employing the isolator 1100 as described above, the following advantageous effects can be achieved.

Since the hydrogen peroxide which has been converted into mists by the mist generation unit 1020 is gasified by the vaporizing unit 1020, the gasification of hydrogen peroxide in the vaporizing unit 1220 is conducted promptly. Consequently, the quantity of heat used in the vaporizing unit can be reduced as compared with the conventional practice. Thus, the time duration required for the gasification of hydrogen peroxide can be shortened and consequently the process of sterilization as a whole can be shortened. As a result, the work efficiency in the isolator 1100 can be improved.

After a predetermined amount of hydrogen peroxide solution has been supplied into the cup 1214 by the pump 1264, whether the internal temperature of the piping 1224 has been reached to a predetermined temperature or not is determined. This can indicate the fact that the remaining amount of hydrogen peroxide solution in the cup 1214 is zero. As a result, the temperature of the heater 1222 can be completely regulated and the consumption of energy in the isolator 1100 can be minimized. Also, the process can be promptly switched from the sterilization process to the replacement process immediately after the remaining amount of hydrogen peroxide solution in the cup 1214 becomes empty. Hence, wasting time can be significantly reduced and the time spent in the process of sterilization as a whole can be shortened.

### (Sterilizing material supply apparatus according to a first modification)

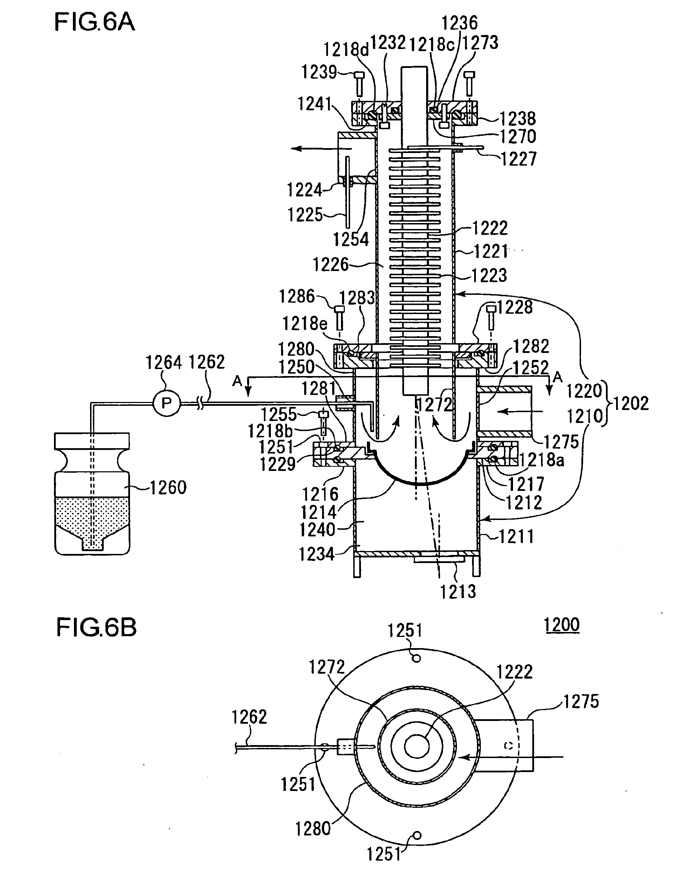

A description is now given of the sterilizing material apparatus 1200 of a first modification, with reference to FIGS. 6A and FIG. 6B. FIG. 6A is a schematic diagram showing the sterilizing supply apparatus 1200 according to the first modification. FIG. 6B is a cross-sectional view taken along the line A-A of FIG. 6A.

The basic structure of the modifications to the sterilizing material apparatus 1200 is similar to that described in the first embodiment. Thus the same structural components as those of the first embodiment is omitted as appropriate and a description is hereinbelow given centering around the difference therefrom.

In the sterilizing material supply apparatus 1200 according to the first modification, the heating pipe 1221 and the holding member 1211 are assembled together by way of a connecting member 1280 (connector). The diameter of the connecting member 1280 is identical to that of the holding member 1211 and is larger than that of the heating pipe 1221.

A flange 1281 is formed around the lower periphery of the connecting member 1280. The outside diameter of the flange 1281 is the same as that of the partition member 1212 and the flange 1216.

The flange 1216, the partition member 1212 and the flange 1218 are provided with screw holes 1251 in predetermined positions. The mist generation unit 1210 and the connecting member 1280 are assembled by screwing screws 1255 into the screw holes 1251 with the 0-rings 1218a and 1218b fitted in the grooves 1217 and 1229, respectively. Airtightness between the flange 1216 and the partition member 1212 is enhanced by the O-ring 1218a. Also, airtightness between the flange 1281 and the partition member 1212 is enhanced by the O-ring 1218b.

In this modification, the opening 1250 and the opening 1252 are located in positions opposite to each other in the lower side surface of the connecting member 1280.

A flange 1282 is formed around the top periphery of the connecting member 1280. The top surface of the flange 1282 is provided with a groove 1283, into which an O-ring 1218a is fitted, and a notch 1285, into which an end of an internal pipe 1272 inserted into the connection member 1280 is fitted. The diameter of the internal pipe 1272 is the same as that of the heating pipe 1221, and the internal pipe 1272 serves as an extending member of the heating pipe 1221.

The flange 1282 is fastened around the lower periphery of the heating pipe 1221 by means of the flange 1228 and a screw 1286. In this case, the O-ring 1218e enhances airtightness between the flange 1282 and the flange 1228.

In the sterilizing material supply apparatus 1200 according to the first modification as configured above, the inside of the connecting member 1280 is of a double structure due to the internal pipe 1272. Thus, in a space between the internal pipe 1272 and the connecting member 1280, the hydrogen peroxide solution is converted into droplets downwardly facing the cup 1214 from the piping 1262. In a space between the internal pipe 1272 and the connecting member 1280, a carrier gas supplied through the piping 1275 flows downward to the cup 1214. It is desirable that the lowest end of the internal pipe 1272 is located below the lowest end (the lowest point of the inside diameter) of an opening (the carrier gas supply port) through which the carrier gas enters.

On the other hand, inside the internal pipe 1272, the hydrogen peroxide solution atomized by the mist generation unit 1210 flows upwardly together with the carrier gas.
Then the thus atomized hydrogen peroxide solution flows into the heating pipe 1221 by way of the internal pipe 1272.

In this manner, the inside of the connecting member 1280 is of a double structure by the use of the internal pipe 1272. Thus, the flow of the carrier gas is downward in the space between the internal pipe 1272 and the connecting member 1280, whereas it is upward inside the internal pipe 1272 because it changes its direction to the opposite on the outer circumference of the cup 1214. As a result, the water column can be stably formed on the surface of the hydrogen peroxide solution stored by the cup 1214. Also, since the lowest end of the internal pipe 1272 is located below the lowest end of the opening 1252 through which the carrier gas enters, the occurrence of a disturbance in the flow of the aforementioned carrier gas can be minimized.

### (Sterilizing material supply apparatus according to a second modification)

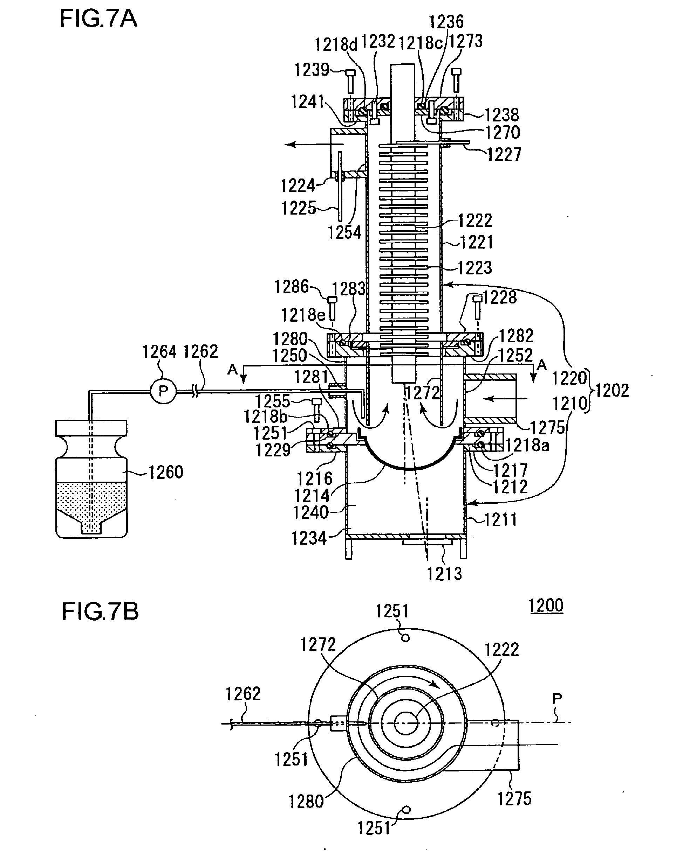

A description is now given of the sterilizing material apparatus 1200 of a second modification, with reference to FIGS. 7A and FIG. 7B. FIG. 7A is a schematic diagram showing the sterilizing supply apparatus 1200 according to the second modification. FIG. 7B is a cross-sectional view taken along the line A-A of FIG. 7A.

The structure of the sterilizing material supply apparatus 1200 according to the second modification is basically the same as that of the first modification and therefore a description is hereinbelow given of differences therefrom.

In the sterilizing material supply apparatus 1200 according to the second modification, the center of the piping 1275 is displaced from a line P passing through the center of the heater 1222 (or the center of the connecting member 1280), as shown in FIG. 7B. By implementing this arrangement, the flow of the carrier gas is downward in the space between the internal pipe 1272 and the connecting member 1280, and the flow direction thereof is clockwise as shown in the cross-sectional view of FIG. 7B. Thus, when the carrier gas folds back on the outer circumference of the cup 1214, the flow thereof becomes an upward flow of a spiral nature. As a result, the water column can be more stably formed on the surface of the hydrogen peroxide solution stored by the cup 1214.

Moreover, the sterilizing supply apparatus 1200 according to the second embodiment may also be configured as follows (Structure 1 to Structure 3), for example:

### Structure 1:

1. A sterilizing material supply apparatus comprising: -
   a mist generation unit including:
   a propagation fluid holding unit for holding a fluid
      through which ultrasonic vibration generated by an ultrasonic vibrator attached to a bottom of the propagation fluid holding unit propagates; and
   a receptacle for holding a hydrogen peroxide solution which is a raw material for sterilizing material,
   the receptacle being attached onto the propagation fluid holding unit in such a manner as to cover an upper opening of the propagation fluid holding unit, and
   a bottom of the receptacle projecting toward the ultrasonic vibrator; and
   a vaporizing unit configured to heat and vaporize the hydrogen peroxide solution atomized by the mist generation unit wherein the vaporizing unit is installed upright above the mist generation unit and a lower opening thereof communicates with the receptacle,
   the vaporizing unit including:
   a carrier gas supply port into which a carrier gas
      carrying the hydrogen peroxide solution atomized thereby flows through a lower side face of the vaporizing unit;
   a heating pipe, disposed on top thereof, having a hydrogen peroxide supply port for supplying hydrogen peroxide, together with the carrier gas, to the exterior;
   and
   a heater disposed inside the heating pipe,
wherein the hydrogen peroxide solution atomized by the
mist generation unit is fed to the vaporizing unit using the carrier gas, and a hydrogen peroxide gas, vaporized by the vaporizing unit, together with the carrier gas is supplied to the exterior.

### Structure 2:

2. A sterilizing material supply apparatus according to Structure 1, further comprising a connection member configured to connect the mist generation unit to the vaporizing unit, the connection member having the carrier gas supply port on a side face thereof
   wherein a diameter of the connection member is greater than that of the heating pipe, and
   in the connection member a lower end of the heating pipe extends further downward from a lower end of the carrier gas supply port.

### Structure 3:

3. A sterilizing material supply apparatus according to Structure 1 or Structure 2, wherein a direction normal to the center of vibrator surface of the ultrasonic vibrator faces the receptacle at a tilt relative to the vertical direction.
   Moreover, the isolator 1100 according to the second embodiment may be configured as follows (Structure 4), for example,:

### Structure 4:

4. An isolator having a workroom in which a work involving biologically-derived materials is performed, the isolator comprising:
   a mist generation unit including:
   a propagation fluid holding unit for holding a fluid
      through which ultrasonic vibration generated by an ultrasonic vibrator attached to a bottom of the propagation fluid holding unit propagates; and
   a receptacle for holding a hydrogen peroxide solution which is a raw material for sterilizing material,
   the receptacle being attached to the propagation fluid holding unit in such a manner as to cover an upper opening of the propagation fluid holding unit, and
   a bottom of the receptacle projecting toward the ultrasonic vibrator; and
   a vaporizing unit configured to heat and vaporize the hydrogen peroxide solution atomized by the mist generation unit, wherein the vaporizing unit is installed upright above the mist generation unit and a lower opening thereof communicates with the receptacle,
   the vaporizing unit including:
      a carrier gas supply port into which a carrier gas carrying the hydrogen peroxide solution atomized thereby flows through a lower side face of the vaporizing unit;
      a heating pipe, disposed on top thereof, having a hydrogen peroxide supply port for supplying hydrogen peroxide, together with the carrier gas, to the exterior; and
      a heater disposed inside the heating pipe,
wherein the hydrogen peroxide solution atomized by the mist generation unit is fed to the vaporizing unit using the carrier gas, and a hydrogen peroxide gas, vaporized by the vaporizing unit, together with the carrier gas is supplied to the exterior.

The present invention is not limited to the above-described embodiments and modifications only, and it is understood by those skilled in the art that various modifications such as changes in design may be made based on their knowledge and the embodiments added with such modifications are also within the scope of the present invention.

For example, in the above-described first embodiment, the valves to be used are not limited to on-off valves, but they may be three-way valves. Also, an opening/closing detection sensor may be provided at the door 30, and the arrangement may be such that at least the second sterilization treatment is not performed when the open state of the door 30 is being detected. Such an arrangement may prevent the inflow of the sterilizing material into the workroom 10 through the door 30 being accidentally open when an attempt is made to sterilize an article in the sterilization chamber 40 while work is going on in the workroom 10. Also, the arrangement may be such that when the open state of the door 30 is being detected, the first sterilization treatment and the second sterilization treatment are not performed, and when the closed state of the door 30 is being detected, the third sterilization treatment is not performed. Such an arrangement may prevent the inflow of the sterilizing material into the sterilization chamber 40 or the workroom 10 through the door 30 being accidentally open when, for instance, an attempt is made to sterilize the workroom 10 or the sterilization chamber 40 by conducting the first sterilization treatment or the second sterilization treatment, which may otherwise lead to an inadequate sterilization due to a low concentration of the sterilizing material. Also, this may prevent the situation in which when an attempt is made to sterilize both of the workroom 10 and the sterilization chamber 40 by conducting the third sterilization treatment, sterilization of only one of the workroom 10 and the sterilization chamber 40 is done because the door 30 is closed. Similarly, an opening/closing detection sensor may be provided at the front door 12 or the carry-in door 58 to control the execution of sterilization treatments according to the results of sensor detection.

Also, the sterilizing material supply apparatus 1200 according to the second embodiment and its first or second modification may be used as the sterilizing material supply unit 200 according to the first embodiment.

## Claims

1. An isolator (1) comprising:
a workroom (10) in which a work involving biologically-derived materials is performed, said workroom including a first sterilizing material supply port (16), and a first gas supply port (18) and a first gas discharge port (20) provided with particulate trap filters (24, 26), respectively;
a sterilization chamber (40) for communicating with said workroom (10) via a door (30) in which an article carried into said workroom is sterilized, said sterilization chamber including a second sterilizing material supply port (42), and a second gas supply port (44) and a second gas discharge port (46) provided with particulate trap filters (50, 52), respectively;
a sterilizing material supply unit (200) for supplying a sterilizing material to said workroom (10) and said sterilization chamber (40);
a first gas supply path (64) for connecting the exterior to the first gas supply port (18);
a second gas supply path (66) for connecting the exterior to the second gas supply port (44);
a first gas discharge path (68) for connecting the first gas discharge port (20) to the exterior;
a second gas discharge path (70) for connecting the second gas discharge port (46) to the exterior; and
a control unit (300) for controlling the switching of flow paths for the sterilizing material and the gas taken in from the exterior;
a first sterilizing material supply path (60) for connecting said sterilizing material supply unit (200) to the first sterilizing material supply port (16) without passing the particulate trap filter (24,26) ; and
a second sterilizing material supply path (62) for connecting said sterilizing material supply unit (200) to the second sterilizing material supply port (42) ; without passing the particulate trap filter (50,52);
**characterized in that**
the workroom (10) further comprises a first sterilizing material discharge port (110), and the sterilization chamber (40) further comprises a second sterilizing material discharge port (112),
wherein a first circulating path (114) connects the first sterilizing material discharge port (110) to said sterilizing material supply unit (200) without passing said particulate trap filter (24, 26); and
wherein a second circulating path (116) connects the second sterilizing material discharge port (112) to said sterilizing material supply unit (200) without passing said particulate trap filter (50, 52).

2. An isolator (1) according to Claim 1, wherein a first sterilization treatment and a second sterilization treatment are executable independently of each other,
the control unit (300) being configured to execute the first sterilization treatment such that the sterilizing material is supplied to said workroom (10) through said first sterilizing material supply path (60) when the door (30) is closed, and
the sterilizing material supplied into said workroom (10) is circulated in the order of said first circulating path (114), said sterilizing material supply unit (200), said first sterilizing material supply path (60) and said workroom (10), and is then discharged to said first gas discharge path (68) from said workroom (10); and
the control unit (300) being further configured to execute the second sterilization treatment such that the sterilizing material is supplied to said sterilization chamber (40) through said second sterilizing material supply path (62) when the door (30) is closed, and
the sterilizing material supplied into said sterilization chamber (40) is circulated in the order of said second circulating path (116), said sterilizing material supply unit (200), said second sterilizing material supply path (62) and said sterilization chamber (40), and is then discharged to said second gas discharge path (70) from said sterilization chamber (40).

3. An isolator (1) according to Claim 1 or Claim 2, wherein the control unit (300) is configured to execute a third sterilization treatment in which the sterilizing material is supplied to said workroom (10) and said sterilization chamber (40) from said first sterilizing material supply path (60) or said second sterilizing material supply path (62) when the door (30) is open, and
the sterilizing material is sent to said sterilizing material supply unit (200) from said second circulating path (116) when said first sterilizing material supply path (60) is used, or
the sterilizing material is sent thereto from said first circulating path (114) when said second sterilizing material supply path (62) is used.

4. An isolator (1) according to Claim 3, wherein the control unit (300) is further configured to execute the third sterilization treatment such that the sterilizing material is supplied to said workroom (10) and said sterilization chamber (40) through said first sterilizing material supply port (60), and
sending out the sterilizing material to said sterilizing material supply unit (200) through said second circulating path (116) and sending out the sterilizing material thereto through said first circulating path (114) are conducted in parallel with each other or in a switchable manner.

5. An isolator (1) according to Claim 2, further comprising:
a first filter circulating path (122), disposed exterior to said workroom (10), for connecting the first gas supply port (18) to the first gas discharge port (20); and
a second filter circulating path (124), disposed exterior to said sterilization chamber (40), for connecting the second gas supply port (44) to the second gas discharge port (46),
wherein, in the first sterilization treatment, the sterilizing material supplied to said workroom (10) is circulated with predetermined timing through said first filter circulating path (122), and
wherein, in the second sterilization treatment, the sterilizing material supplied to said sterilization chamber (40) is circulated with predetermined timing through said second filter circulating path (124).

6. An isolator (1) according to Claim 5, wherein said first filter circulating path (122) includes a first decomposition treatment unit path (128) that passes through a sterilizing material decomposition treatment unit (132) having a catalyst for decomposing the sterilizing material, the sterilizing material decomposition treatment unit (132) being configured to reduce the concentration of the sterilizing material to be discharged outside,
wherein said second filter circulating path (124) includes a second decomposition treatment unit path (136) that passes through a sterilizing material decomposition treatment unit (140) having a catalyst for decomposing the sterilizing material, the sterilizing material decomposition treatment unit (140) being configured to reduce the concentration of the sterilizing material to be discharged outside,
wherein, in the first sterilization treatment, the sterilizing material supplied to said workroom (10) is circulated with predetermined timing through the first decomposition treatment unit path (128), and
wherein, in the second sterilization treatment, the sterilizing material supplied to said sterilization chamber (40) is circulated with predetermined timing through the second decomposition treatment unit path (136).

7. An isolator (1) according to any one of Claim 1 to Claim 6, said sterilizing material supply unit (200) comprising:
a mist generation unit (1210) including:
a propagation fluid holding unit (1211) for holding a fluid (1240) through which ultrasonic vibration generated by an ultrasonic vibrator (1213) attached to a bottom of the holding unit (1211) propagates; and
a receptacle (1214) for holding a hydrogen peroxide solution which is a raw material for sterilizing material,
the receptacle (1214) being attached onto the propagation fluid holding unit (1211) in such a manner as to cover an upper opening of the propagation fluid holding unit (1211), and
a bottom of the receptacle (1214) projecting toward the ultrasonic vibrator (1213); and
a vaporizing unit (1220) configured to heat and vaporize the hydrogen peroxide solution atomized by the mist generation unit (1210), wherein said vaporizing unit (1220) is installed upright above said mist generation unit (1210) and a lower opening thereof communicates with the receptacle (1214),
the vaporizing unit (1220) including:
a carrier gas supply port (1252) into which a carrier gas carrying the hydrogen peroxide solution atomized thereby flows through a lower side face of said vaporizing unit
a heating pipe (1221), disposed on top thereof, having a hydrogen peroxide supply port (1254) for supplying hydrogen peroxide, together with the carrier gas, to the exterior; and
a heater (1222) disposed inside the heating pipe (1221),
wherein the hydrogen peroxide solution atomized by the mist generation unit (1210) is fed to the vaporizing unit (1220) using the carrier gas, and a hydrogen peroxide gas, vaporized by the vaporizing unit (1220), together with the carrier gas is supplied to the exterior.

8. An isolator (1) according to Claim 7, said sterilizing material supply apparatus further comprising a connection member (1280) configured to connect the mist generation unit (1210) to the vaporizing unit (1220), the connection member (1280) having the carrier gas supply port (1252) on a side face thereof
wherein a diameter of the connection member (1280) is greater than that of the heating pipe (1221), and
in the connection member (1280) a lower end of the heating pipe (1221) extends further downward from a lower end of the carrier gas supply port (1252).

9. An isolator (1) according to any one of Claim 7 or Claim 8, wherein a direction normal to a center of vibrator surface of the ultrasonic vibrator (1213) faces the receptacle (1214) at a tilt relative to a vertical direction.

## Patentansprüche

1. Isolator (1), aufweisend:
einen Arbeitsraum (10), in dem eine Arbeit durchgeführt wird, die biologisch abgeleitete Materialien beinhaltet, wobei der Arbeitsraum jeweils eine erste Sterilisierungsmaterialzuleitungsöffnung (16) sowie eine erste Gaszuleitungsöffnung (18) und eine erste Gasableitungsöffnung (20) enthält, die mit Teilchenauffangfiltern (24, 26) versehen sind;
eine Sterilisierungskammer (40) zur Verbindung mit dem Arbeitsraum (10) über eine Tür (30), in der ein Gegenstand, der in den Arbeitsraum gebracht wird, sterilisiert wird, wobei die Sterilisierungskammer jeweils eine zweite Sterilisierungsmaterialzuleitungsöffnung (42) sowie eine zweite Gaszuleitungsöffnung (44) und eine zweite Gasableitungsöffnung (46) enthält, die mit Teilchenauffangfiltern (50, 52) versehen sind;
eine Sterilisierungsmaterialzuleitungseinheit (200) zum Zuleiten eines Sterilisierungsmaterials zu dem Arbeitsraum (10) und der Sterilisierungskammer (40);
einen ersten Gaszuleitungspfad (64) zum Anschließen der Außenseite an die erste Gaszuleitungsöffnung (18);
einen zweiten Gaszuleitungspfad (66) zum Anschließen der Außenseite an die zweite Gaszuleitungsöffnung (44);
einen ersten Gasableitungspfad (68) zum Anschließen der ersten Gasableitungsöffnung (20) an die Außenseite;
einen zweiten Gasableitungspfad (70) zum Anschließen der zweiten Gasableitungsöffnung (46) an die Außenseite; und
eine Steuereinheit (300) zum Steuern des Umschaltens von Strömungspfaden für das Sterilisierungsmaterial und das Gas, das von der Außenseite eingeführt wird;
einen ersten Sterilisierungsmaterialzuleitungspfad (60) zum Anschließen der Sterilisierungsmaterialzuleitungseinheit (200) an die erste Sterilisierungsmaterialzuleitungsöffnung (16) ohne das Teilchenauffangfilter (24, 26) zu passieren; und
einen zweiten Sterilisierungsmaterialzuleitungspfad (62) zum Anschließen der Sterilisierungsmaterialzuleitungseinheit (200) an die zweite Sterilisierungsmaterialzuleitungsöffnung (42) ohne das Teilchenauffangfilter (50, 52) zu passieren;
**dadurch gekennzeichnet, dass**
der Arbeitsraum (10) des Weiteren eine erste Sterilisierungsmaterialableitungsöffnung (110) aufweist und die Sterilisierungskammer (40) des Weiteren eine zweite Sterilisierungsmaterialableitungsöffnung (112) aufweist;
wobei ein erster Zirkulierungspfad (114) die erste Sterilisierungsmaterialableitungsöffnung (110) an die Sterilisierungsmaterialzuleitungseinheit (200) anschließt, ohne das Teilchenauffangfilter (24, 26) zu passieren; und
wobei ein zweiter Zirkulationspfad (116) die zweite Sterilisierungsmaterialableitungsöffnung (112) an die Sterilisierungsmaterialzuleitungseinheit (200) anschließt, ohne das Teilchenauffangfilter (50, 52) zu passieren.

2. Isolator (1) nach Anspruch 1, wobei eine erste Sterilisierungsbehandlung und eine zweite Sterilisierungsbehandlung unabhängig voneinander durchführbar sind,
wobei die Steuereinheit (300) zum derartigen Ausführen der ersten Sterilisierungsbehandlung gestaltet ist, dass das Sterilisierungsmaterial dem Arbeitsraum (10) durch den ersten Sterilisierungsmaterialzuleitungspfad (60) zugeleitet wird, wenn die Tür (30) geschlossen ist, und
das Sterilisierungsmaterial, das in den Arbeitsraum (10) zugeleitet wird, in der Reihenfolge erster Zirkulierungspfad (114), Sterilisierungsmaterialzuleitungseinheit (200), erster Sterilisierungsmaterialzuleitungspfad (60) und Arbeitsraum (10) zirkuliert wird und dann aus dem Arbeitsraum (10) an den ersten Gasableitungspfad (68) abgegeben wird; und
die Steuereinheit (300) des Weiteren zum derartigen Ausführen der zweiten Sterilisierungsbehandlung gestaltet ist, dass das Sterilisierungsmaterial der Sterilisierungskammer (40) durch den zweiten Sterilisierungsmaterialzuleitungspfad (62) zugeleitet wird, wenn die Tür (30) geschlossen ist, und
das Sterilisierungsmaterial, das in die Sterilisierungskammer (40) zugeleitet wird, in der Reihenfolge zweiter Zirkulierungspfad (116), Sterilisierungsmaterialzuleitungseinheit (200), zweiter Sterilisierungsmaterialzuleitungspfad (62) und Sterilisierungskammer (40) zirkuliert wird und dann aus der Sterilisierungskammer (40) an den zweiten Gasableitungspfad (70) abgegeben wird.

3. Isolator (1) nach Anspruch 1 oder Anspruch 2, wobei die Steuereinheit (300) zum Ausführen einer dritten Sterilisierungsbehandlung gestaltet ist, in der das Sterilisierungsmaterial von dem ersten Sterilisierungsmaterialzuleitungspfad (60) oder dem zweiten Sterilisierungsmaterialzuleitungspfad (62) zu dem Arbeitsraum (10) und der Sterilisierungskammer (40) zugeleitet wird, wenn die Tür (30) offen ist, und
das Sterilisierungsmaterial von dem zweiten Zirkulierungspfad (116) zu der Sterilisierungsmaterialzuleitungseinheit (200) gesendet wird, wenn der erste Sterilisierungsmaterialzuleitungspfad (60) verwendet wird, oder
das Sterilisierungsmaterial von dem ersten Zirkulierungspfad (114) dorthin gesendet wird, wenn der zweite Sterilisierungsmaterialzuleitungspfad (62) verwendet wird.

4. Isolator (1) nach Anspruch 3, wobei die Steuereinheit (300) des Weiteren zum derartigen Ausführen der dritten Sterilisierungsbehandlung gestaltet ist, dass das Sterilisierungsmaterial durch den ersten Sterilisierungsmaterialzuleitungspfad (60) zu dem Arbeitsraum (10) und der Sterilisierungskammer (40) geleitet wird, und
das Senden des Sterilisierungsmaterials durch den zweiten Zirkulierungspfad (116) zu der Sterilisierungsmaterialzuleitungseinheit (200) und das Senden des Sterilisierungsmaterials durch den ersten Zirkulierungspfad (114) zu dieser parallel oder auf umschaltbare Weise ausgeführt werden.

5. Isolator (1) nach Anspruch 2, des Weiteren aufweisend:
einen ersten Filterzirkulierungspfad (122), der außerhalb des Arbeitsraums (10) angeordnet ist, zum Anschließen der ersten Gaszuleitungsöffnung (18) an die erste Gasableitungsöffnung (20); und
einen zweiten Filterzirkulierungspfad (124), der außerhalb der Sterilisierungskammer (40) angeordnet ist, zum Anschließen der zweiten Gaszuleitungsöffnung (44) an die zweite Gasableitungsöffnung (46),
wobei in der ersten Sterilisierungsbehandlung das Sterilisierungsmaterial, das dem Arbeitsraum (10) zugeleitet wird, mit einer vorbestimmten Zeitsteuerung durch den ersten Filterzirkulierungspfad (122) zirkuliert wird, und
wobei in der zweiten Sterilisierungsbehandlung das Sterilisierungsmaterial, das der Sterilisierungskammer (40) zugeleitet wird, mit einer vorbestimmten Zeitsteuerung durch den zweiten Filterzirkulierungspfad (124) zirkuliert wird.

6. Isolator (1) nach Anspruch 5, wobei der erste Filterzirkulierungspfad (122) einen ersten Zersetzungsbehandlungseinheitspfad (128) enthält, der durch eine Sterilisierungsmaterial-Zersetzungsbehandlungseinheit (132) geht, die einen Katalysator für eine Zersetzung des Sterilisierungsmaterials aufweist, wobei die Sterilisierungsmaterial-Zersetzungsbehandlungseinheit (132) zur Verringerung der Konzentration des Sterilisierungsmaterials gestaltet ist, das nach außen abzugeben ist,
wobei der zweite Filterzirkulierungspfad (124) einen zweiten Zersetzungsbehandlungseinheitspfad (136) enthält, der durch eine Sterilisierungsmaterial-Zersetzungsbehandlungseinheit (140) geht, die einen Katalysator für eine Zersetzung des Sterilisierungsmaterials aufweist, wobei die Sterilisierungsmaterial-Zersetzungsbehandlungseinheit (140) zur Verringerung der Konzentration des Sterilisierungsmaterials gestaltet ist, das nach außen abzugeben ist,
wobei in der ersten Sterilisierungsbehandlung das Sterilisierungsmaterial, das dem Arbeitsraum (10) zugeleitet wird, mit einer vorbestimmten Zeitsteuerung durch den ersten Zersetzungsbehandlungseinheitspfad (128) zirkuliert wird, und
wobei in der zweiten Sterilisierungsbehandlung das Sterilisierungsmaterial, das der Sterilisierungskammer (40) zugeleitet wird, mit einer vorbestimmten Zeitsteuerung durch den zweiten Zersetzungsbehandlungseinheitspfad (136) zirkuliert wird.

7. Isolator (1) nach einem von Anspruch 1 bis Anspruch 6, wobei die Sterilisierungsmaterialzuleitungseinheit (200) Folgendes aufweist:
eine Nebelerzeugungseinheit (1210), enthaltend:
eine Ausbreitungsfluidaufnahmeeinheit (1211) zum Aufnehmen eines Fluids (1240), durch das sich Ultraschallvibration ausbreitet, die von einem Ultraschallvibrator (1213) erzeugt wird, der am Boden der Aufnahmeeinheit (1211) befestigt ist; und
einen Behälter (1214) zum Aufnehmen einer Wasserstoffperoxidlösung, die ein Rohmaterial für Sterilisierungsmaterial ist,
wobei der Behälter (1214) derart an der Ausbreitungsfluidaufnahmeeinheit (1211) befestigt ist, dass er eine obere Öffnung der Ausbreitungsfluidaufnahmeeinheit (1211) bedeckt, und
ein Boden des Behälters (1214) zum Ultraschallvibrator (1213) vorragt; und
eine Verdampfungseinheit (1220), die zum Erwärmen und Verdampfen der Wasserstoffperoxidlösung gestaltet ist, die von der Nebelerzeugungseinheit (1210) zerstäubt wird, wobei die Verdampfungseinheit (1220) aufrecht über der Nebelerzeugungseinheit (1210) eingebaut ist und ihre untere Öffnung mit dem Behälter (1214) in Verbindung steht,
wobei die Verdampfungseinheit (1220) Folgendes enthält:
eine Trägergaszuleitungsöffnung (1252), in die ein Trägergas, das die dadurch zerstäubte Wasserstoffperoxidlösung trägt, durch eine untere Seitenfläche der Verdampfungseinheit strömt,
ein Heizrohr (1221), das an ihrer Oberseite angeordnet ist, mit einer Wasserstoffperoxidzuleitungsöffnung (1254) zum Zuleiten von Wasserstoffperoxid gemeinsam mit dem Trägergas zur Außenseite; und
eine Heizvorrichtung (1222), die im Inneren des Heizrohres (1221) angeordnet ist,
wobei die Wasserstoffperoxidlösung, die von der Nebelerzeugungseinheit (1210) zerstäubt wurde, der Verdampfungseinheit (1220) mit Hilfe des Trägergases zugeführt wird, und ein Wasserstoffperoxidgas, das von der Verdampfungseinheit (1220) verdampft wurde, gemeinsam mit dem Trägergas der Außenseite zugeleitet wird.

8. Isolator (1) nach Anspruch 7, wobei die Sterilisierungs material zuleitungs vorrichtung des Weiteren ein Verbindungselement (1280) aufweist, das zum Anschluss der Nebelerzeugungseinheit (1210) an die Verdampfungseinheit (1220) gestaltet ist, wobei das Verbindungselement (1280) an einer Seitenfläche die Trägergaszuleitungsöffnung (1252) aufweist,
wobei ein Durchmesser des Verbindungselements (1280) größer als jener des Heizrohres (1221) ist, und
sich in dem Verbindungselement (1280) ein unteres Ende des Heizrohres (1221) von einem unteren Ende der Trägergaszuleitungsöffnung (1252) weiter nach unten erstreckt.

9. Isolator (1) nach einem von Anspruch 7 oder Anspruch 8, wobei eine Richtung normal zu einem Mittelpunkt einer Vibratoroberfläche des Ultraschallvibrators (1213) dem Behälter (1214) mit einer Neigung relativ zu einer vertikalen Richtung zugewandt ist.

## Revendications

1. Isolateur (1) comprenant :
une salle de travail (10) dans laquelle un travail impliquant des substances d'origine biologique est exécuté, ladite salle de travail comprenant un premier orifice de fourniture de substance de stérilisation (16), et un premier orifice de fourniture de gaz (18) et un premier orifice d'évacuation de gaz (20) respectivement dotés de filtres à particules (24, 26) ;
une chambre de stérilisation (40) pour communiquer avec ladite salle de travail (10) par l'intermédiaire d'une porte (30) dans laquelle un article emporté dans ladite salle de travail est stérilisé, ladite chambre de stérilisation comprenant un deuxième orifice de fourniture de substance de stérilisation (42), et un deuxième orifice de fourniture de gaz (44) et un deuxième orifice d'évacuation de gaz (46) respectivement dotés de filtres à particules (50, 52) ;
une unité de fourniture de substance de stérilisation (200) pour fournir une substance de stérilisation dans ladite salle de travail (10) et ladite chambre de stérilisation (40) ;
une première voie de fourniture de gaz (64) pour raccorder l'extérieur au premier orifice de fourniture de gaz (18) ;
une deuxième voie de fourniture de gaz (66) pour raccorder l'extérieur au deuxième orifice de fourniture de gaz (44) ;
une première voie d'évacuation de gaz (68) pour raccorder le premier orifice d'évacuation de gaz (20) à l'extérieur ;
une deuxième voie d'évacuation de gaz (70) pour raccorder le deuxième orifice d'évacuation de gaz (46) à l'extérieur ; et
une unité de commande (300) pour commander la commutation des voies d'écoulement de la substance de stérilisation et du gaz entrant de l'extérieur ;
une première voie de fourniture de substance de stérilisation (60) pour raccorder ladite unité de fourniture de substance de stérilisation (200) au premier orifice de fourniture de substance de stérilisation (16) sans passer par le filtre à particules (24, 26) ; et
une deuxième voie de fourniture de substance de stérilisation (62) pour raccorder ladite unité de fourniture de substance de stérilisation (200) au deuxième orifice de fourniture de substance de stérilisation (42) sans passer par le filtre à particules (50, 52) ;
**caractérisé en ce que**
la salle de travail (10) comprend en outre un premier orifice d'évacuation de substance de stérilisation (110), et la chambre de stérilisation (40) comprend en outre un deuxième orifice d'évacuation de substance de stérilisation (112),
dans lequel une première voie de circulation (114) raccorde le premier orifice d'évacuation de substance de stérilisation (110) à ladite unité de fourniture de substance de stérilisation (200) sans passer par ledit filtre à particules (24, 26) ; et
dans lequel une deuxième voie de circulation (116) raccorde le deuxième orifice d'évacuation de substance de stérilisation (112) à ladite unité de fourniture de substance de stérilisation (200) sans passer par ledit filtre à particules (50, 52).

2. Isolateur (1) selon la revendication 1, dans lequel un premier traitement de stérilisation et un deuxième traitement de stérilisation peuvent être exécutés indépendamment l'un de l'autre,
l'unité de commande (300) étant configurée pour exécuter le premier traitement de stérilisation, de sorte que la substance de stérilisation soit fournie dans ladite salle de travail (10) à travers ladite première voie de fourniture de substance de stérilisation (60) quand la porte (30) est fermée, et
la substance de stérilisation fournie dans ladite salle de travail (10) est mise en circulation, dans cet ordre, dans ladite première voie de circulation (114), dans ladite unité de fourniture de substance de stérilisation (200), dans ladite première voie de fourniture de substance de stérilisation (60) et dans ladite salle de travail (10), et est ensuite évacuée de ladite salle de travail (10) à travers ladite première voie d'évacuation de gaz (68) ; et
l'unité de commande (300) étant en outre configurée pour exécuter le deuxième traitement de stérilisation, de sorte que la substance de stérilisation soit fournie dans ladite chambre de stérilisation (40) à travers ladite deuxième voie de fourniture de substance de stérilisation (62) quand la porte (30) est fermée, et
la substance de stérilisation fournie dans ladite chambre de stérilisation (40) est mise en circulation, dans cet ordre, dans ladite deuxième voie de circulation (116), dans ladite unité de fourniture de substance de stérilisation (200), dans ladite deuxième voie de fourniture de substance de stérilisation (62) et dans ladite chambre de stérilisation (40), et est ensuite évacuée de ladite chambre de stérilisation (40) à travers ladite deuxième voie d'évacuation de gaz (70).

3. Isolateur (1) selon la revendication 1 ou la revendication 2, dans lequel l'unité de commande (300) est configurée pour exécuter un troisième traitement de stérilisation dans lequel la substance de stérilisation est fournie dans ladite salle de travail (10) et ladite chambre de stérilisation (40) à partir de ladite première voie de fourniture de substance de stérilisation (60) ou de ladite deuxième voie de fourniture de substance de stérilisation (62) quand la porte (30) est ouverte, et
la substance de stérilisation est envoyée vers ladite unité de fourniture de substance de stérilisation (200) à partir de ladite deuxième voie de circulation (116) quand ladite première voie de fourniture de substance de stérilisation (60) est utilisée, ou
la substance de stérilisation y est envoyée à partir de ladite première voie de circulation (114) quand ladite deuxième voie de fourniture de substance de stérilisation (62) est utilisée.

4. Isolateur (1) selon la revendication 3, dans lequel l'unité de commande (300) est configurée en outre pour exécuter le troisième traitement de stérilisation, de sorte que la substance de stérilisation soit fournie dans ladite salle de travail (10) et ladite chambre de stérilisation (40) à travers ladite première voie de fourniture de substance de stérilisation (60), et
l'envoi de la substance de stérilisation vers ladite unité de fourniture de substance de stérilisation (200) à travers ladite deuxième voie de circulation (116) et l'envoi de la substance de stérilisation à travers ladite première voie de circulation (114) sont réalisés en parallèle l'un avec l'autre ou d'une manière commutable.

5. Isolateur (1) selon la revendication 2, comprenant en outre :
une première voie de circulation de filtration (122), disposée à l'extérieur de ladite salle de travail (10), pour raccorder le premier orifice de fourniture de gaz (18) au premier orifice d'évacuation de gaz (20) ; et
une deuxième voie de circulation de filtration (124), disposée à l'extérieur de ladite chambre de stérilisation (40), pour raccorder le deuxième orifice de fourniture de gaz (44) au deuxième orifice d'évacuation de gaz (46),
dans lequel, dans le premier traitement de stérilisation, la substance de stérilisation fournie dans ladite salle de travail (10) est mise en circulation avec une durée prédéterminée à travers ladite première voie de circulation de filtration (122), et
dans lequel, dans le deuxième traitement de stérilisation, la substance de stérilisation fournie dans ladite chambre de stérilisation (40) est mise en circulation avec une durée prédéterminée à travers ladite deuxième voie de circulation de filtration (124).

6. Isolateur (1) selon la revendication 5, dans lequel ladite première voie de circulation de filtration (122) comprend une première voie d'unité de traitement par décomposition (128) qui passe à travers une unité de traitement par décomposition de substance de stérilisation (132) comportant un catalyseur pour décomposer la substance de stérilisation, l'unité de traitement par décomposition de substance de stérilisation (132) étant configurée pour réduire la concentration de la substance de stérilisation à évacuer vers l'extérieur,
dans lequel ladite deuxième voie de circulation de filtration (124) comprend une deuxième voie d'unité de traitement par décomposition (136) qui passe à travers une unité de traitement par décomposition de substance de stérilisation (140) comportant un catalyseur pour décomposer la substance de stérilisation, l'unité de traitement par décomposition de substance de stérilisation (140) étant configurée pour réduire la concentration de la substance de stérilisation à évacuer vers l'extérieur,
dans lequel, dans le premier traitement de stérilisation, la substance de stérilisation fournie dans ladite salle de travail (10) est mise en circulation avec une durée prédéterminée à travers la première voie d'unité de traitement par décomposition (128), et
dans lequel, dans le deuxième traitement de stérilisation, la substance de stérilisation fournie dans ladite chambre de stérilisation (40) est mise en circulation avec une durée prédéterminée à travers la deuxième voie d'unité de traitement par décomposition (136).

7. Isolateur (1) selon l'une quelconque des revendications 1 à 6, ladite unité de fourniture de substance de stérilisation (200) comprenant :
une unité de génération de brume (1210) comportant :
une unité de conservation de fluide de propagation (1211) pour contenir un fluide (1240) à travers lequel des vibrations ultrasonores générées par un vibrateur ultrasonore (1213) fixé au fond de l'unité de conservation (1211) se propagent ; et
un réceptacle (1214) pour contenir une solution de peroxyde d'hydrogène qui est une matière première pour la substance de stérilisation,
le réceptacle (1214) étant fixé sur l'unité de conservation de fluide de propagation (1211) de façon à recouvrir une ouverture supérieure de l'unité de conservation de fluide de propagation (1211), et
un fond du réceptacle (1214) faisant saillie vers le vibrateur ultrasonore (1213) ; et
une unité de vaporisation (1220) configurée pour chauffer et vaporiser la solution de peroxyde d'hydrogène atomisée par l'unité de génération de brouillard (1210), dans lequel ladite unité de vaporisation (1220) est installée verticalement au-dessus de ladite unité de génération de brume(1210) et une ouverture inférieure de celle-ci communique avec le réceptacle (1214),
l'unité de vaporisation (1220) comportant :
un orifice de fourniture de gaz vecteur (1252) dans lequel un gaz vecteur portant la solution de peroxyde d'hydrogène ainsi atomisée s'écoule à travers une face latérale inférieure de ladite unité de vaporisation,
un tuyau chauffant (1221), disposé sur sa partie supérieure, doté d'un orifice de fourniture de peroxyde d'hydrogène (1254) pour fournir du peroxyde d'hydrogène, conjointement avec le gaz vecteur, vers l'extérieur ; et
un appareil de chauffage (1222) disposé à l'intérieur du tuyau chauffant (1221),
dans lequel la solution de peroxyde d'hydrogène atomisée par l'unité de génération de brume (1210) est introduite dans l'unité de vaporisation (1220) au moyen du gaz vecteur, et du peroxyde d'hydrogène gazeux, vaporisé par l'unité de vaporisation (1220), conjointement avec le gaz vecteur, est fourni vers l'extérieur.

8. Isolateur (1) selon la revendication 7, ledit appareil de fourniture de substance de stérilisation comprenant en outre un organe de raccordement (1280) configuré pour raccorder l'unité de génération de brume (1210) à l'unité de vaporisation (1220), l'organe de raccordement (1280) ayant l'orifice de fourniture de gaz vecteur (1252) sur l'une de ses faces latérales,
dans lequel un diamètre de l'organe de raccordement (1280) est supérieur à celui du tuyau chauffant (1221), et
dans l'organe de raccordement (1280), une extrémité inférieure du tuyau chauffant (1221) s'étend davantage vers le bas à partir d'une extrémité inférieure de l'orifice de fourniture de gaz vecteur (1252).

9. Isolateur (1) selon la revendication 7 ou la revendication 8, dans lequel une direction perpendiculaire à un centre de la surface vibrante du vibrateur ultrasonore (1213) fait face au réceptacle (1214) à une inclinaison par rapport à une direction verticale.
